(19) Europäisches Patentamt
European Patent Office
Office européen des brevets

(11) EP 4 299 690 A1

(12) # EUROPEAN PATENT APPLICATION
published in accordance with Art. 153(4) EPC

(43) Date of publication:
**03.01.2024 Bulletin 2024/01**

(21) Application number: **22759328.2**

(22) Date of filing: **04.02.2022**

(51) International Patent Classification (IPC):
***C09K 9/02*** *(2006.01)* ***G01J 1/50*** *(2006.01)*

(52) Cooperative Patent Classification (CPC):
**C09K 9/02; G01J 1/50**

(86) International application number:
**PCT/JP2022/004340**

(87) International publication number:
**WO 2022/181288 (01.09.2022 Gazette 2022/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.02.2021 JP 2021030713**
**20.08.2021 JP 2021134875**

(71) Applicant: **FUJIFILM Corporation**
**Tokyo 106-8620 (JP)**

(72) Inventors:
- **IKEDA Kimi**
**Fujinomiya-shi, Shizuoka 418-8666 (JP)**
- **ARIOKA Daisuke**
**Fujinomiya-shi, Shizuoka 418-8666 (JP)**

(74) Representative: **Hoffmann Eitle**
**Patent- und Rechtsanwälte PartmbB**
**Arabellastraße 30**
**81925 München (DE)**

(54) **UV RADIATION SENSITIVE MEMBER AND UV RADIATION SENSITIVE KIT**

(57) The present invention provides an ultraviolet-sensing member in which it is easy to determine whether an irradiation amount which inactivates the novel coronavirus has been irradiated, and an ultraviolet-sensing kit. In the ultraviolet-sensing member of the present invention, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 mJ/$cm^2$, and using a spectrophotometer Spectrolino (GretagMacbeth AG), optical densities of predetermined colors are measured with the ultraviolet-sensing member before the light irradiation and the ultraviolet-sensing member after the light irradiation, the optical densities show a predetermined value.

FIG. 1

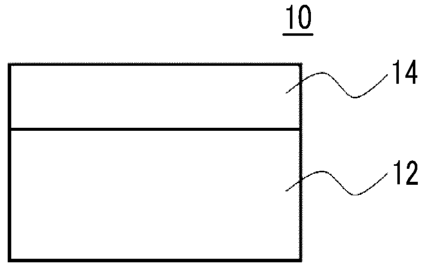


EP 4 299 690 A1

## Description

## BACKGROUND OF THE INVENTION

1. Field of the Invention

The present invention relates to an ultraviolet-sensing member and an ultraviolet-sensing kit.

2. Description of the Related Art

A measurement of an amount of ultraviolet irradiation has been carried out in various fields. Specific examples thereof include a measurement of an amount of ultraviolet irradiation to an object to be irradiated in a curing reaction of an ultraviolet curing resin, and a measurement of an amount of ultraviolet irradiation to an object to be irradiated in an ultraviolet sterilization of food or the like.

As the measurement of the amount of ultraviolet irradiation, for example, JP2015-191001A discloses a method of using "UV label" (UV-H manufactured by NiGK Corporation), and WO2017/158943A discloses a method of using "UV scale" (manufactured by FUJIFILM Corporation).

On the other hand, in recent years, infection with novel coronavirus (COVID-19) has been a major social problem.

Under these circumstances, in Hiroki, Kitagawa et al., "Effectiveness of 222-nm ultraviolet light on disinfecting SARS-CoV-2 surface contamination", American Journal of Infection Control, Internet (https://www.sciencedirect.com/science/article/pii/S0196655320308099), an inactivation effect of the novel coronavirus using ultraviolet rays with a wavelength of 222 nm has been reported. More specifically, in Hiroki, Kitagawa et al., "Effectiveness of 222-nm ultraviolet light on disinfecting SARS-CoV-2 surface contamination", American Journal of Infection Control, Internet (https://www.sciencedirect.com/science/article/pii/S0196655320308099), it has been reported that 99.7% of the novel coronavirus is inactivated by radiating ultraviolet rays with a wavelength of 222 nm at an illuminance of 0.1 $mW/cm^2$ for 30 seconds.

## SUMMARY OF THE INVENTION

As disclosed in Hiroki, Kitagawa et al., "Effectiveness of 222-nm ultraviolet light on disinfecting SARS-CoV-2 surface contamination", American Journal of Infection Control, Internet (https://www.sciencedirect.com/science/article/pii/S0196655320308099), since ultraviolet rays with a wavelength of 222 nm are effective in inactivating the novel coronavirus, for example, by irradiating ultraviolet rays with a wavelength of 222 nm to a member which is touched by an unspecified number of people, such as a doorknob and a touch panel, it is possible to prevent the infection with the novel coronavirus. In this case, it is desirable that it is possible to easily measure whether or not an irradiation amount of ultraviolet rays which achieves the inactivation of the novel coronavirus is applied to a predetermined position.

In a case of measuring, using a known UV label and UV scale in the related art, whether the amount of light with a wavelength of 222 nm, which inactivates the novel coronavirus infection, is irradiated, the present inventors cannot determine the irradiation amount because almost no change in tint is observed with the UV label and UV scale. More specifically, even in a case where the UV label and the UV scale are irradiated with ultraviolet rays with a wavelength of 222 nm at an integrated illuminance of 3 $mJ/cm^2$, almost no change in tint is observed.

In view of the above-described circumstances, an object of the present invention is to provide an ultraviolet-sensing member in which it is easy to determine whether an irradiation amount which inactivates the novel coronavirus has been irradiated.

Another object of the present invention is to provide an ultraviolet-sensing kit.

As a result of intensive studies to achieve the above-described objects, the present inventors have found that the above-described objects can be achieved by the following configurations, and have completed the present invention.

(1) An ultraviolet-sensing member,

in which, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 $mJ/cm^2$,

using a spectrophotometer Spectrolino (GretagMacbeth AG), values of yellow optical densities, magenta optical densities, and cyan optical densities are measured with the ultraviolet-sensing member before the light irradiation and the ultraviolet-sensing member after the light irradiation, and

a value of a cyan optical density of the ultraviolet-sensing member before the light irradiation is denoted as C1, a value of a cyan optical density of the ultraviolet-sensing member after the light irradiation is denoted

as C2, a value of a yellow optical density of the ultraviolet-sensing member before the light irradiation is denoted as Y1, a value of a yellow optical density of the ultraviolet-sensing member after the light irradiation is denoted as Y2, a value of a magenta optical density of the ultraviolet-sensing member before the light irradiation is denoted as M1, and a value of a magenta optical density of the ultraviolet-sensing member after the light irradiation is denoted as M2,

any one of a difference between C1 and C2, a difference between Y1 and Y2, or a difference between M1 and M2 is 0.20 or more.

(2) An ultraviolet-sensing member,
in which, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 $mJ/cm^2$, a color difference $\Delta E$ between before the light irradiation and after the light irradiation is 20.0 or more.
(3) An ultraviolet-sensing member,
in which, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 $mJ/cm^2$, a difference between an integrated value 1 of an absorbance of the ultraviolet-sensing member before the light irradiation in a wavelength range of 450 to 700 nm, which is obtained by a method 1 described later, and an integrated value 2 of an absorbance of the ultraviolet-sensing member after the light irradiation in the wavelength range of 450 to 700 nm, which is obtained by a method 2 described later, is 18.0 or more.
(4) The ultraviolet-sensing member according to any one of (1) to (3),
in which the ultraviolet-sensing member is a sheet-like ultraviolet-sensing member.
(5) The ultraviolet-sensing member according to any one of (1) to (4),
in which the ultraviolet-sensing member includes an ultraviolet-sensing layer containing a color-forming agent.
(6) The ultraviolet-sensing member according to (5),
in which a content of the color-forming agent in the ultraviolet-sensing layer is 0.140 $g/m^2$ or less per unit area of the ultraviolet-sensing layer.
(7) The ultraviolet-sensing member according to (5) or (6),

in which the color-forming agent is selected from the group consisting of a color-forming agent which forms color by being oxidized and a color-forming agent which forms color by action of acid, and
the ultraviolet-sensing layer contains at least one photoactivator selected from the group consisting of a photooxidant and a photoacid generator.

(8) The ultraviolet-sensing member according to (7),

in which the color-forming agent is a color-forming agent which forms color by action of acid,
the ultraviolet-sensing layer contains a photoacid generator, and
a mass ratio of a content of the photoactivator to a content of the color-forming agent is more than 1.00.

(9) The ultraviolet-sensing member according to (7) or (8),
in which the photoactivator includes a compound represented by General Formula (6) described later.
(10) The ultraviolet-sensing member according to (7) or (8),
in which the color-forming agent includes any one structure selected from the group consisting of a lactone ring, a lactam ring, a sultone ring, a sultine ring, a ring-opened body of these rings, and an azobenzene structure.
(11) The ultraviolet-sensing member according to any one of (1) to (6),
in which the ultraviolet-sensing member includes an ultraviolet-sensing layer containing a microcapsule which contains a photoactivator, a color-forming agent, and a solvent having a heteroatom.
(12) The ultraviolet-sensing member according to (11),

in which a capsule wall of the microcapsule contains one or more resins selected from the group consisting of a polyurea having an aliphatic ring, a polyurethane urea having an aliphatic ring, and a polyurethane having an aliphatic ring, and
a peak surface area proportion X obtained by a peak surface area proportion calculation method X described later is 30% or less.

(13) An ultraviolet-sensing kit comprising:
the ultraviolet-sensing member according to any one of (1) to (12).

According to the present invention, it is possible to provide an ultraviolet-sensing member in which it is easy to determine whether an irradiation amount which inactivates the novel coronavirus has been irradiated.
According to the present invention, it is possible to provide an ultraviolet-sensing kit.

## BRIEF DESCRIPTION OF THE DRAWINGS

Fig. 1 is a schematic cross-sectional view showing an example of a first embodiment of the ultraviolet-sensing member according to the present invention.

## DESCRIPTION OF THE PREFERRED EMBODIMENTS

Hereinafter, the present invention will be described in detail.
The description of the configuration requirements described below is made on the basis of representative embodiments of the present invention, but it should not be construed that the present invention is limited to those embodiments.
In the present specification, the numerical ranges shown using "to" indicate ranges including the numerical values described before and after "to" as the lower limit value and the upper limit value.
In addition, regarding numerical ranges that are described stepwise in the present specification, an upper limit value or a lower limit value described in a numerical range may be replaced with an upper limit value or a lower limit value of another stepwise numerical range. In addition, in the numerical range described in the present specification, an upper limit value and a lower limit value described in a certain numerical range may be replaced with values shown in Examples.
In addition, in the present specification, a solid content means a component forming a composition layer formed of a composition, and in a case where the composition contains a solvent (for example, organic solvent, water, and the like), the solid content means all components excluding the solvent. In addition, in a case where the components are components which form a composition layer, the components are considered to be solid contents even in a case where the components are liquid components.
In addition, in the present specification, ultraviolet rays mean light having a wavelength range of 10 to 400 nm.
In addition, in the present specification, (meth)acrylic means "at least one of acrylic or methacrylic".
In addition, in the present specification, "boiling point" means a boiling point at a standard atmospheric present.
An embodiment A of the ultraviolet-sensing member according to the present invention is an ultraviolet-sensing member in which, in a case where, using a krypton chloride (KrCl) excimer lamp as a light source, the ultraviolet-sensing member is irradiated with light through a filter which substantially shields light having a wavelength of 230 to 300 nm until an irradiation amount of light having a wavelength of 222 nm reaches 3 $mJ/cm^2$, using a spectrophotometer Spectrolino (GretagMacbeth AG), values of cyan optical densities, magenta optical densities, and yellow optical densities are measured with the ultraviolet-sensing member before the light irradiation and the ultraviolet-sensing member after the light irradiation, and a value of a cyan optical density of the ultraviolet-sensing member before the light irradiation is denoted as C1, a value of a cyan optical density of the ultraviolet-sensing member after the light irradiation is denoted as C2, a value of a yellow optical density of the ultraviolet-sensing member before the light irradiation is denoted as Y1, a value of a yellow optical density of the ultraviolet-sensing member after the light irradiation is denoted as Y2, a value of a magenta optical density of the ultraviolet-sensing member before the light irradiation is denoted as M1, and a value of a magenta optical density of the ultraviolet-sensing member after the light irradiation is denoted as M2, any one of a difference between C1 and C2, a difference between Y1 and Y2, or a difference between M1 and M2 is 0.20 or more.
An embodiment B of the ultraviolet-sensing member according to the present invention is an ultraviolet-sensing member in which, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 $mJ/cm^2$, a color difference $\Delta E$ between before the light irradiation and after the light irradiation is 20.0 or more.
An embodiment C of the ultraviolet-sensing member according to the present invention is an ultraviolet-sensing member in which, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 $mJ/cm^2$, a difference between an integrated value 1 of an absorbance of the ultraviolet-sensing member before the light irradiation in a wavelength range of 450 to 700 nm, which is obtained by a method 1 described later, and an integrated value 2 of an absorbance of the ultraviolet-sensing member after the light irradiation in the wavelength range of 450 to 700 nm, which is obtained by a method 2

described later, is 18.0 or more.

In the ultraviolet-sensing members according to the embodiments A to C of the present invention, in a case of being irradiated with ultraviolet rays with a wavelength of 222 nm at an integrated illuminance of 3 mJ/cm$^2$, there is a change in tint, so that it is easy to determine whether the irradiation amount which inactivates the novel coronavirus has been irradiated.

In addition, it has been found that the ultraviolet-sensing members according to the embodiments A to C of the present invention are less likely to cause so-called fogging, in which color is formed by unexpected light such as a fluorescent lamp.

In the following, first, the above-described characteristics of the ultraviolet-sensing members according to the embodiments A to C of the present invention will be described in detail.

First, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 mJ/cm$^2$. From the viewpoint of easy handling, a size of the ultraviolet-sensing member to be irradiated with light is preferably a size of a length of 5 mm to 10 m in a vertical direction and a length of 5 mm to 300 mm in a horizontal direction.

The filter which substantially shields light having a wavelength of 230 to 300 nm means a filter which shields 70% to 100% of the light having a wavelength of 230 to 300 nm. In other words, the above-described filter is a filter having a maximum transmittance of 30% or less in the wavelength range of 230 to 300 nm. As such a filter, a chemical filter or a filter containing a dielectric is usually used.

As an ultraviolet irradiation device in which the KrCl excimer lamp is used as the light source and the filter which substantially shields light having a wavelength of 230 to 300 nm is provided, an ultraviolet irradiation device Care 222 (registered trademark) available from Ushio Inc. may be used. In the Care 222 (registered trademark), a lamp in which a main wavelength is ultraviolet rays with a wavelength of 222 nm, which is suitable for sterilization, is combined with a filter that limits light to a wavelength range (wavelength of 200 to 230 nm) which is harmless to the human body. Therefore, in a case where light irradiation is performed using the Care 222 (registered trademark), light having a wavelength of 222 nm is mainly irradiated.

Illuminance and irradiation time in the case of the irradiation are not particularly limited, and the irradiation amount can be set to 3 mJ/cm$^2$ by adjusting a distance between the light source and the ultraviolet-sensing member and the irradiation time.

In addition, using a known ultraviolet-measuring device (for example, Handheld Light Meter UIT2400 (manufactured by Ushio Inc.)), it may be measured whether or not a predetermined irradiation amount has been applied to the ultraviolet-sensing member.

Next, in the embodiment A, using a spectrophotometer Spectrolino (GretagMacbeth AG), values of cyan optical densities, magenta optical densities, and yellow optical densities are measured with the ultraviolet-sensing member before the light irradiation and the ultraviolet-sensing member after the light irradiation.

More specifically, in a case of using the spectrophotometer Spectrolino (GretagMacbeth AG), measurement conditions are set as follows.

· Density status: DIN
· D65/2°
· No filter

In a case where the ultraviolet-sensing member before the light irradiation and the ultraviolet-sensing member after the light irradiation are to be measured, by measuring optical densities (OD) in each of Cyan (C) mode, Magenta (M) mode, and Yellow (Y) mode, a value (Y value) of the cyan optical density, a value (M value) of the magenta optical density, and a value (Y value) of the yellow optical density are measured respectively. It is preferable that the measurement of the optical density (OD) of the ultraviolet-sensing member after the light irradiation is carried out within 1 hour after the light irradiation.

Next, in a case where a value of a cyan optical density of the ultraviolet-sensing member before the light irradiation is denoted as C1, a value of a cyan optical density of the ultraviolet-sensing member after the light irradiation is denoted as C2, a value of a yellow optical density of the ultraviolet-sensing member before the light irradiation is denoted as Y1, a value of a yellow optical density of the ultraviolet-sensing member after the light irradiation is denoted as Y2, a value of a magenta optical density of the ultraviolet-sensing member before the light irradiation is denoted as M1, and a value of a magenta optical density of the ultraviolet-sensing member after the light irradiation is denoted as M2, a difference between C1 and C2, a difference between Y1 and Y2, and a difference between M1 and M2 are calculated.

The difference between C1 and C2 is a value obtained by subtracting the smaller one of C1 and C2 from the larger one thereof. In a case where the values of C1 and C2 are the same, the difference between the two is 0.

The difference between Y1 and Y2 is a value obtained by subtracting the smaller one of Y1 and Y2 from the

larger one thereof. In a case where the values of Y1 and Y2 are the same, the difference between the two is 0.

The difference between M1 and M2 is a value obtained by subtracting the smaller one of M1 and M2 from the larger one thereof. In a case where the values of M1 and M2 are the same, the difference between the two is 0.

Any one of the difference between C1 and C2, the difference between Y1 and Y2, or the difference between M1 and M2, which are obtained by the above-described procedure, is 0.20 or more, preferably 0.20 to 1.50 and more preferably 0.26 to 1.10.

Among these, from the viewpoint that it is easier to determine whether the irradiation amount which inactivates the novel coronavirus has been irradiated (hereinafter, also simply referred to as "viewpoint that the effect of the present invention is more excellent"), it is preferable that any one of the difference between C1 and C2 or the difference between M1 and M2 is 0.20 or more.

The difference between C1 and C2 is preferably 0.20 or more, more preferably 0.20 to 1.50, and still more preferably 0.26 to 1.10.

The value of C1 is often 0.00 to 0.10, preferably 0.00 to 0.05.

The value of C2 is preferably 0.20 or more, and more preferably 0.20 to 1.50.

The difference between M1 and M2 is preferably 0.20 or more, more preferably 0.20 to 1.50, and still more preferably 0.26 to 1.10.

The value of M1 is often 0.00 to 0.10, preferably 0.00 to 0.05.

The value of M2 is preferably 0.20 or more, and more preferably 0.20 to 1.50.

The difference between Y1 and Y2 is preferably 0.20 or more, more preferably 0.20 to 1.50, and still more preferably 0.26 to 1.10.

The value of Y1 is often 0.00 to 0.10, preferably 0.00 to 0.05.

The value of Y2 is preferably 0.20 or more, and more preferably 0.20 to 1.50.

In addition, in the embodiment B, a color difference $\Delta E$ between the ultraviolet-sensing member before the light irradiation and the ultraviolet-sensing member after the light irradiation is measured.

More specifically, in a case of using the spectrophotometer Spectrolino (GretagMacbeth AG), measurement conditions are set as follows.

- Irradiation type: D65
- Observation field: 2°
- Density standard: ANSI STATUS A

Using the above-described spectrophotometer, the ultraviolet-sensing member before the light irradiation and the ultraviolet-sensing member after the light irradiation are set as objects to be measured, and a lightness L*, a chromaticity a*, and a chromaticity b* defined by the CIE1976L*a*b* color system are measured. It is preferable that the measurement of the lightness L*, chromaticity a*, and chromaticity b* of the ultraviolet-sensing member after the light irradiation is carried out within 1 hour after the light irradiation.

Next, ΔL* which is a difference between the lightness L* of the ultraviolet-sensing member before the light irradiation (hereinafter, also referred to as "lightness L*1") and the lightness L* of the ultraviolet-sensing member after the light irradiation (hereinafter, also referred to as "lightness L*2"), Δa* which is a difference between the chromaticity a* of the ultraviolet-sensing member before the light irradiation (hereinafter, also referred to as "chromaticity a*1") and the chromaticity a* of the ultraviolet-sensing member after the light irradiation (hereinafter, also referred to as "chromaticity a*2"), and Δb* which is a difference between the chromaticity b* of the ultraviolet-sensing member before the light irradiation (hereinafter, also referred to as "chromaticity b*1") and the chromaticity b* of the ultraviolet-sensing member after the light irradiation (hereinafter, also referred to as "chromaticity b*2") are obtained, and the color difference $\Delta E$ is calculated from the following expression.

$$\Delta E = \{(\Delta L^*)^2 + (\Delta a^*)^2 + (\Delta b^*)^2\}^{1/2}$$

The color difference $\Delta E$ is 20.0 or more, and from the viewpoint that the effect of the present invention is more excellent, it is preferably 24.0 to 130 and more preferably 35.0 to 100.

The difference between the lightness L*1 and the lightness L*2 is a value obtained by subtracting the smaller one of the lightness L*1 and the lightness L*2 from the larger one thereof. In a case where the values of the lightness L*1 and the lightness L*2 are the same, the difference between the two is 0.

In addition, the difference between the chromaticity a*1 and the chromaticity a*2 is a value obtained by subtracting the smaller one of the chromaticity a*1 and the chromaticity a*2 from the larger one thereof. In a case where the values of the chromaticity a*1 and the chromaticity a*2 are the same, the difference between the two is 0.

In addition, the difference between the chromaticity b*1 and the chromaticity b*2 is a value obtained by subtracting the smaller one of the chromaticity b*1 and the chromaticity b*2 from the larger one thereof. In a case where the values of the chromaticity b*1 and the chromaticity b*2 are the same, the difference between the two is 0.

In addition, in the embodiment C, an integrated value 1 of an absorbance of the ultraviolet-sensing member before the light irradiation in a wavelength range of 450 to 700 nm, which is obtained by a method 1, and an integrated value 2 of an absorbance of the ultraviolet-sensing member after the light irradiation in the wavelength range of 450 to 700 nm, which is obtained by a method 2, are obtained. It is preferable that the measurement of the integrated value 2 of the absorbance of the ultraviolet-sensing member after the light irradiation in the wavelength range of 450 to 700 nm is carried out within 1 hour after the light irradiation.

Method 1: a reflection spectrum of the ultraviolet-sensing member before the light irradiation is measured to obtain a reflection spectrum in which a horizontal axis is a wavelength and a vertical axis is an absorbance, and then the integrated value 1 of the absorbance is obtained by integrating the absorbance for each 1 nm from a wavelength of 450 nm to a wavelength of 700 nm in the reflection spectrum

Method 2: a reflection spectrum of the ultraviolet-sensing member after the light irradiation is measured to obtain a reflection spectrum in which a horizontal axis is a wavelength and a vertical axis is an absorbance, and then the integrated value 2 of the absorbance is obtained by integrating the absorbance for each 1 nm from a wavelength of 450 nm to a wavelength of 700 nm in the reflection spectrum

An ultraviolet-visible spectrophotometer (UV-2700, Shimadzu Corporation) is used as a device for measuring the reflection spectrum in the methods 1 and 2 described above. The measurement range is in a range of 350 to 750 nm, and the absorbance is measured for each 1 nm.

In the above-described device and measurement conditions, the absorbance is measured by a diffuse-reflect measurement to obtain a reflection spectrum in which a horizontal axis is the wavelength and a vertical axis is the absorbance, the integrated value of the absorbance is obtained by integrating the absorbance for each 1 nm from the wavelength of 450 nm to the wavelength of 700 nm in the reflection spectrum.

In a case where an object to be measured is the ultraviolet-sensing member before the light irradiation, the integrated value of the obtained absorbance is the integrated value 1, and in a case where an object to be measured is the ultraviolet-sensing member after the light irradiation, the integrated value of the obtained absorbance is the integrated value 2.

Next, a difference between the obtained integrated value 1 and the obtained integrated value 2 is calculated.

The above-described difference is 18.0 or more, and from the viewpoint that the effect of the present invention is more excellent, it is preferably 20.0 to 200 and more preferably 29.0 to 150.

The difference between the integrated value 1 and the integrated value 2 is a value obtained by subtracting the smaller one of the integrated value 1 and the integrated value 2 from the larger one thereof. In a case where the values of the integrated value 1 and the integrated value 2 are the same, the difference between the two is 0.

A form of the ultraviolet-sensing members according to the embodiments A to C of the present invention is not particularly limited, and may be a sheet-like shape, and various shapes such as a block shape, for example, a rectangular parallelepiped shape, a cylindrical shape, and the like can be used. Among these, a sheet-like ultraviolet-sensing member, that is, an ultraviolet-sensing sheet is suitably used.

In addition, as the shape of the sheet-like ultraviolet-sensing member, various shapes such as a square shape, a rectangular shape, a circular shape, an elliptical shape, a polygonal shape other than a quadrangular shape, for example, a hexagonal shape and the like, and an amorphous shape can be used. In addition, the sheet-like ultraviolet-sensing member may have a long shape.

The ultraviolet-sensing member may be applied on another member. In a case of being applied on another member, the ultraviolet-sensing member may be attached through an adhesive layer such as a pressure sensitive adhesive and an adhesive, or may be manufactured as a part of another member. Another member is not particularly limited, and examples thereof include a business card, a name tag, a mask, a cloth product (for example, a shirt), a case (for example, a smartphone case), and a paper product (for example, a notebook, a calender, and the like).

Hereinafter, materials (particularly, a color-forming agent and a photoactivator) which can be contained in the ultraviolet-sensing members according to the embodiments A to C of the present invention will be described in detail. In the following, the ultraviolet-sensing members according to the embodiments A to C of the present invention will be collectively referred to as "ultraviolet-sensing member".

(Color-forming agent)

The ultraviolet-sensing member preferably includes an ultraviolet-sensing layer containing a color-forming agent. A specific configuration of the ultraviolet-sensing member will be described in detail later.

Here, the "color-forming" of the "color-forming agent" is a concept including coloration, discoloration, and decolorization. The coloration includes coloring from a state of being substantially colorless (a state in which it is colorless or exhibits a light color) by action of acid, oxidation, light irradiation, or the like. In addition, the discoloration includes a

change in color from a specific colored state to another colored state or colorless state (for example, a change from yellow color to red color) due to an influence of action of acid, oxidation, light irradiation, or the like. The "decolorization" means a change from a specific colored state to a state of being substantially colorless (a state in which it is colorless or exhibits a light color).

The type of the color-forming agent is not particularly limited, and examples thereof include a color-forming agent which forms color by being oxidized, a color-forming agent which forms color by action of acid, and a color-forming agent which forms color by action of light. Among these, a color-forming agent which forms color by being oxidized or a color-forming agent which forms color by action of acid is preferable, and a color-forming agent which forms color by action of acid is more preferable.

The color-forming agent may be used alone or in combination of two or more kinds thereof.

The color-forming agent is preferably a color-forming agent including any one structure selected from the group consisting of a lactone ring, a lactam ring, a sultone ring, a sultine ring, a ring-opened body of these rings, and an azobenzene structure.

As the color-forming agent, a leuco coloring agent or a photochromic coloring agent is preferable, and a leuco coloring agent is more preferable.

The photochromic coloring agent is known as a compound which forms color by being isomerized by the action of light, a compound which forms color by progressing a ring-closing reaction by the action of light, a compound which forms color by progressing a ring-opening reaction by the action of light, or the like, and a known photochromic coloring agent can be used. It is preferable that the photochromic coloring agent has a coloring-decolorizing reaction which reversibly proceeds with energy.

The above-described leuco coloring agent is preferably a compound which forms color in a case of being oxidized from a substantially colorless state (hereinafter, also referred to as "oxidative color-forming leuco coloring agent") or a compound which forms color by the action of acid from a substantially colorless state (hereinafter, also referred to as "acid color-forming leuco coloring agent"). From the viewpoint that it is easy to design the ultraviolet-sensing members of the embodiments A to C, an acid color-forming leuco coloring agent is preferable.

Examples of the leuco coloring agent include a triarylmethanephthalide-based compound, a fluoran-based compound, a phenothiazine-based compound, an indolylphthalide-based compound, an azaindolylphthalide-based compound, a leuco auramine-based compound, a rhodamine lactam-based compound, a triarylmethane-based compound, a diarylmethane-based compound, a triazene-based compound, a spiropyran-based compound, a thiazine-based compound, and a fluorene-based compound.

For details of the above-described compounds, reference can be made to the description of US3445234A, JP1993-257272A (JP-H5-257272A), and paragraphs 0029 to 0034 of WO2009/008248A.

· Oxidative color-forming leuco coloring agent

As one aspect of the oxidative color-forming leuco coloring agent, a compound having one or two hydrogen atoms, which forms color by removing electrons, is preferable. Examples of such an oxidative color-forming leuco coloring agent include (a) aminotriarylmethane, (b) aminoxanthine, (c) aminothioxanthine, (d) amino-9,10-dihydroacridine, (e) aminophenoxazine, (f) aminophenothiazine, (g) aminodihydrophenazine, (h) aminodiphenylmethane, (i) leuco indamine, (j) aminohydrocinnamic acid (cyanethane and leuco methine), (k) hydrazine, (l) leuco indigoid dye, (m) amino-2,3-dihydroanthraquinone, (n) tetrahalo-p,p'-biphenol, (o) 2-(p-hydroxyphenyl)-4,5-diphenylimidazole, and (p) phenethylaniline, which are described in US3445234A. Among the above-described (a) to (p), (a) to (i) form color by losing one hydrogen atom, and (j) to (p) form color by losing two hydrogen atoms.

Among these, aminoarylmethane is preferable, and aminotriarylmethane is more preferable.

As the aminotriarylmethane, a compound represented by Formula (L) or an acid salt thereof is preferable.

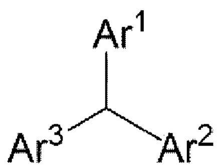

(L)

In the formula, $Ar^1$ represents (A1) a phenyl group having a $R^1R^2N$-substituent at a para position with respect to a bond to a methane carbon atom specified in the formula. $Ar^2$ represents (A1) a phenyl group having a $R^1R^2N$-

substituent at a para position with respect to a bond to a methane carbon atom specified in the formula or (A2) a phenyl group having, at an ortho position with respect to the methane carbon atom specified in the formula, a substituent selected from the group consisting of an alkyl group (preferably, an alkyl group having 1 to 4 carbon atoms), an alkoxy group (preferably, an alkoxy group having 1 to 4 carbon atoms), a fluorine atom, a chlorine atom, and a bromine atom. $R^1$ and $R^2$ each independently represent a hydrogen atom, an alkyl group having 1 to 10 carbon atoms, a 2-hydroxyethyl group, a 2-cyanoethyl group, or a benzyl group.

$Ar^3$ represents the same group as at least one of $Ar^1$ or $Ar^2$, or a group different from $Ar^1$ and $Ar^2$. In a case where $Ar^3$ represents a group different from Ar1 and $Ar^2$, $Ar^3$ represents (B1) a phenyl group which may be substituted with a substituent selected from the group consisting of a lower alkyl group (preferably, an alkyl group having 1 to 4 carbon atoms), a lower alkoxy group (preferably, an alkoxy group having 1 to 4 carbon atoms), a chlorine atom, a diphenylamino group, a cyano group, a nitro group, a hydroxy group, a fluorine atom, a bromine atom, an alkylthio group, an arylthio group, a thioester group, an alkylsulfonic acid group, an arylsulfonic acid group, a sulfonic acid group, a sulfonamide group, an alkylamide group, and an arylamide group; (B2) a naphthyl group which may be substituted with a substituent selected from the group consisting of an amine group, a di-lower alkylamino group, and an alkylamino group; (B3) a pyridyl group which may be substituted with an alkyl group; (B4) a quinolyl group; or (B5) an indolinylidene group which may be substituted with an alkyl group.

In Formula (L), $R^1$ and $R^2$ are each independently preferably a hydrogen atom or an alkyl having 1 to 4 carbon atoms.

In addition, in Formula (L), it is preferable that all of $Ar^1$, $Ar^2$, and $Ar^3$ are the phenyl group having a $R^1R^2N$-substituent at a para position with respect to a bond to a methane carbon atom specified in the formula, and it is more preferable that all of $Ar^1$, $Ar^2$, and $Ar^3$ are the same group thereof.

Specific examples of the oxidative color-forming leuco coloring agent include tris(4-dimethylaminophenyl)methane, tris(4-diethylaminophenyl)methane, bis(4-diethylaminophenyl)-(4-diethylamino-2-methylphenyl)methane, bis(4-diethylamino-2-methylphenyl)-(4-diethylaminophenyl)methane, bis(1-ethyl-2-methylindol-3-yl)-phenylmethane, 2-N-(3-trifluoromethylphenyl)-N-ethylamino-6-diethylamino-9-(2-methoxycarbonylphenyl)xa nthene, 2-(2-chlorophenyl)amino-6-dibutylamino-9-(2-methoxycarbonylphenyl)xanthene, 2-dibenzylamino-6-diethylamino-9-(2-methoxycarbonylphenyl)xanthene, benzo[a]-6-N,N-diethylamino-9,2-methoxycarbonylphenylxanthene, 2-(2-chlorophenyl)-amino-6-dibutylamino-9-(2-methylphenylcarboxamidophenyl)xanthene, 3,6-dimethoxy-9-(2-methoxycarbonyl)-phenylxanthene, benzoyl leuco methylene blue, and 3,7-bis-diethylaminophenoxazine.

· Acid color-forming leuco coloring agent

As one aspect of the acid color-forming leuco coloring agent, a compound which forms color by donating electrons or receiving protons such as an acid is preferable. Specific examples thereof include a compound which has a partial skeleton such as a lactone (cyclic carboxylic acid ester), a lactam (cyclic carboxylic acid amide), a sultone (cyclic sulfonic acid ester), a sultine (cyclic sulfonic acid amide), a spiropyran, an ester, and an amide, in which these partial skeletons are ring-opened or cleaved upon contact with an acid or a proton.

From the viewpoint that it is easy to design the ultraviolet-sensing members of the embodiments A to C, a compound having any one structure selected from the group consisting of a lactone, a lactam, a sultone, a sultine, and a ring-opened body thereof (hereinafter, also referred to as "specific color-forming agent") is preferable.

The specific color-forming agent has a predetermined structure, and the structure can be reversibly changed.

For example, a specific color-forming agent X having the following lactone structure can be a ring-closed body which is substantially colorless and a ring-opened body which is colored. In a case where an acid is supplied to the ring-closed body of the specific color-forming agent X, as shown in the following scheme, a reaction in which the ring is opened by action of the acid ($H^+$) to produce a ring-opened body and a reaction in which the ring is closed by deacidification to produce a ring-closed body proceed reversibly, and higher amounts of the acid shift equilibrium more toward the reaction which produces the ring-opened body. As the equilibrium is biased to the reaction which produces the ring-opened body, color formability is enhanced, and the determination of the irradiation amount is easier. As will be described later, in a case where a mass ratio of a content of the photoactivator to a content of the color-forming agent (photoactivator/color-forming agent (mass ratio)) is more than 1.00, since the amount of acid generated with respect to the specific color-forming agent X increases, the equilibrium tends to be biased in the reaction from the ring-closed body to the ring-opened body, and as a result, the color formability is further enhanced. In addition, the amount of the color-forming agent can be relatively reduced in the mass ratio as described above, and in this case, absorption of light by the specific color-forming agent X itself can be suppressed, and as a result, light absorption by the photoactivator can be enhanced.

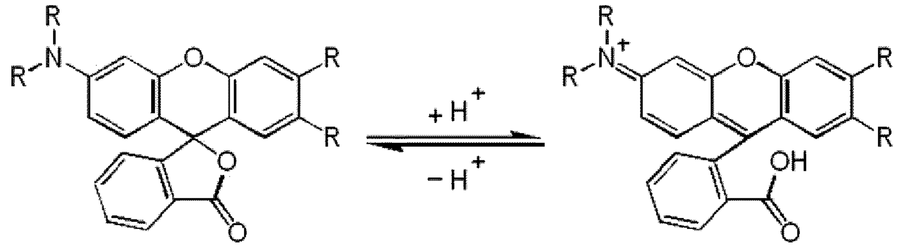

Although the aspect of the compound which is ring-opened by the action of acid has been described above, the compound which is ring-closed by the action of acid may also be used as described below.

As shown in the following scheme, a specific color-forming agent Y having the following sultone structure can be a ring-opened body exhibiting blue color and a ring-closed body exhibiting yellow color. In a case where an acid is supplied to the ring-opened body of the specific color-forming agent Y, as shown in the following scheme, a reaction in which the ring is closed by action of the acid ($H^+$) to produce a ring-closed body and a reaction in which the ring is opened by deacidification to produce a ring-opened body proceed reversibly, and higher amounts of the acid shift equilibrium more toward the reaction which produces the ring-closed body. As the equilibrium is biased to the reaction which produces the ring-closed body, color formability is enhanced, and the determination of the irradiation amount is easier.

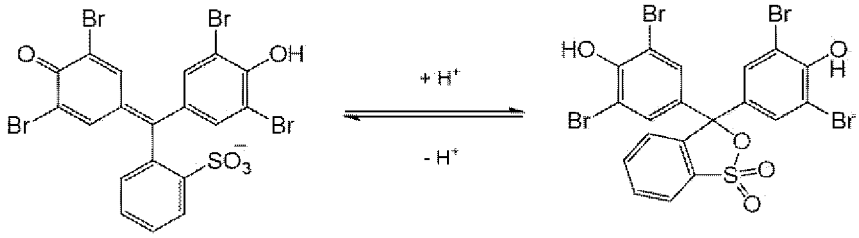

In addition, as shown in the following scheme, a specific color-forming agent Z having the following azobenzene structure can be an azo body exhibiting yellow color and a hydrazone body exhibiting orange to pink color. In a case where an acid is supplied to the azo body of the specific color-forming agent Z, as shown in the following scheme, a reaction in which the hydrazone body is produced by action of the acid ($H^+$) and a reaction in which the azo body is produced by deacidification proceed reversibly, and higher amounts of the acid shift equilibrium more toward the reaction which produces the hydrazone body. As the equilibrium is biased to the reaction which produces the hydrazone body, color formability is enhanced, and the determination of the irradiation amount is easier.

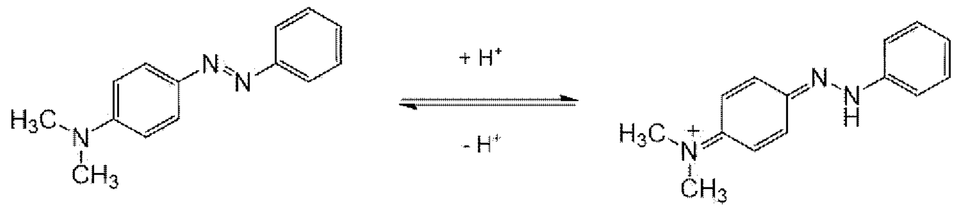

Examples of the above-described specific color-forming agent also include a compound represented by Formula (II) and a ring-opened compound thereof.

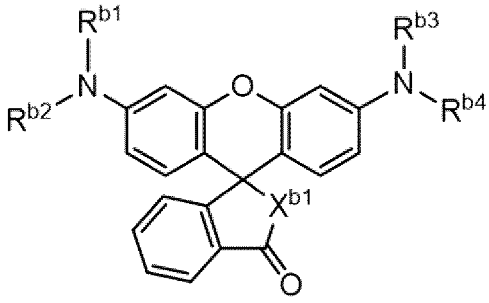

(II)

In Formula (II), $R^{b1}$ to $R^{b3}$ each independently represent a hydrogen atom, an alkyl group which may have a substituent, or an aryl group which may have a substituent. $R^{b2}$ and $R^{b4}$ each independently represent an alkyl group which may have a substituent or an aryl group which may have a substituent. $X^{b1}$ represents $-NR^{b5}-$. $R^{b5}$ represents a hydrogen atom, an alkyl group which may have a substituent, or an aryl group which may have a substituent.

The above-described alkyl group which may have a substituent, represented by $R^{b1}$ to $R^{b5}$, may be linear, branched, or cyclic, and is preferably linear or branched.

The number of carbon atoms in the above-described alkyl group which may have a substituent is preferably 1 to 10 and more preferably 1 to 5.

The above-described aryl group which may have a substituent, represented by $R^{b1}$ to $R^{b5}$, may be monocyclic or polycyclic.

The number of carbon atoms in the above-described aryl group which may have a substituent is preferably 6 to 20.

Examples of the substituent which may be included in the above-described alkyl group which may have a substituent and the above-described aryl group which may have a substituent include a halogen atom, a cyano group, a nitro group, a carboxy group, an alkyl group, an aryl group, and a group obtained by combining these groups.

As $R^{b1}$ to $R^{b4}$, an alkyl group which may have a substituent is preferable, and it is more preferable to have no substituent (unsubstituted alkyl group).

Examples of the specific color-forming agent also include a compound represented by Formula (III) and a ring-opened compound thereof.

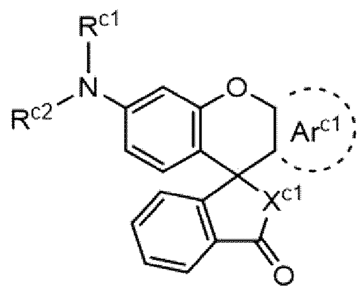

(III)

In Formula (III), $Ar^{c1}$ represents an aromatic ring which may have a substituent. $R^{c1}$ represents a hydrogen atom, an alkyl group which may have a substituent, or an aryl group which may have a substituent. $R^{c2}$ represents an alkyl group which may have a substituent or an aryl group which may have a substituent. $X^{c1}$ represents -O- or $-NR^{c3}-$. $R^{c3}$ represents a hydrogen atom, an alkyl group which may have a substituent, or an aryl group which may have a substituent.

$Ar^{c1}$ represents an aromatic ring which may have a substituent.

The above-described aromatic ring which may have a substituent may be monocyclic or polycyclic.

Examples of the above-described aromatic ring which may have a substituent include an aromatic hydrocarbon ring which may have a substituent and an aromatic heterocyclic ring which may have a substituent, and an aromatic hydrocarbon ring which may have a substituent is preferable.

Examples of the substituent which may be included in the above-described aromatic ring which may have a substituent include an alkyl group, $-NR^{N1}R^{N2}$, an aryl group, and a heteroaryl group, and an alkyl group or $-NR^{N1}R^{N2}$ is

preferable. $R^{N1}$ and $R^{N2}$ each independently represent a hydrogen atom, an alkyl group which may have a substituent, or an aryl group which may have a substituent.

The above-described alkyl group may be linear, branched, or cyclic, and is preferably linear or branched.

The number of carbon atoms in the above-described alkyl group is preferably 1 to 20 and more preferably 1 to 5.

The number of ring members in the above-described aromatic ring which may have a substituent is preferably 6 to 30, more preferably 6 to 18, and still more preferably 6 to 12.

Examples of the above-described aromatic ring which may have a substituent include aromatic hydrocarbon rings such as a benzene ring, a naphthalene ring, an anthracene ring, and a phenanthrene ring, which may have a substituent; and aromatic heterocyclic rings such as an indole ring, a pyrrole ring, a pyrazole ring, a triazole ring, a pyridine ring, a pyrazine ring, a pyrimidine ring, a pyridazine ring, a thiophene ring, a furan ring, a pyran ring, a thiazole ring, an oxazole ring, a selenophene ring, and an imidazole ring, which may have a substituent. Among these, an aromatic hydrocarbon ring which may have a substituent is preferable, and a benzene ring which may have a substituent is more preferable.

$R^{c1}$ represents a hydrogen atom, an alkyl group which may have a substituent, or an aryl group which may have a substituent. $R^{c2}$ represents an alkyl group which may have a substituent or an aryl group which may have a substituent.

Examples of the alkyl group which may have a substituent and the aryl group which may have a substituent, which are represented by $R^{c1}$, include the alkyl group which may have a substituent and the aryl group which may have a substituent, which are represented by $R^{b1}$ to $R^{b5}$ described above.

As $R^{c1}$ and $R^{c2}$, a linear or branched alkyl group which does not have a substituent is preferable.

$X^{c1}$ represents -O- or $-NR^{c3}-$. $R^{c3}$ represents a hydrogen atom, an alkyl group which may have a substituent, or an aryl group which may have a substituent.

Examples of the alkyl group which may have a substituent and the aryl group which may have a substituent, which are represented by $R^{c3}$, include the alkyl group which may have a substituent and the aryl group which may have a substituent, which are represented by $R^{b1}$ to $R^{b5}$ described above.

Examples of the specific color-forming agent include a compound represented by Formula (IV) and a ring-closed compound thereof, and a compound represented by Formula (V).

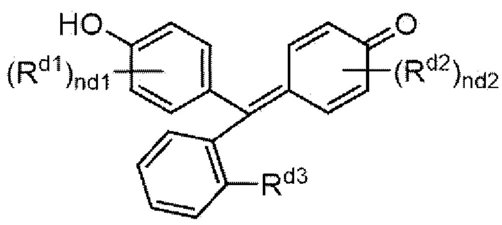

(IV)

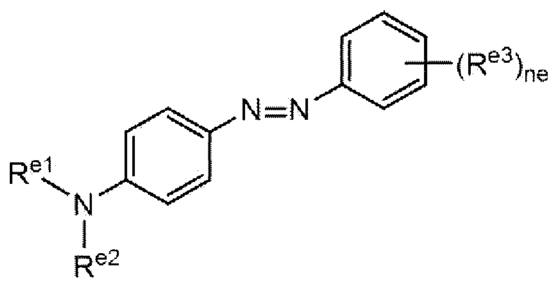

(V)

In Formula (IV), $R^{d1}$ and $R^{d2}$ each independently represent a halogen atom or an alkyl group which may have a substituent. $R^{d3}$ represents $COO^-M_d^+$ or $SO_3^-M_d^+$. $M_d^+$ represents a cation. nd1 and nd2 each independently represent an integer of 0 to 4.

In Formula (V), $R^{e1}$ and $R^{e2}$ each independently represent an alkyl group which may have a substituent. $R^{e3}$ represents $COO^-M_e^+$ or $SO_3^-M_e^+$. $M_e^+$ represents a cation. ne represents 0 or 1.

$R^{d1}$ and $R^{d2}$ each independently represent a halogen atom or an alkyl group which may have a substituent.

The above-described halogen atom is preferably a chlorine atom or a bromine atom.

The above-described alkyl group which may have a substituent may be linear, branched, or cyclic.

The number of carbon atoms in the above-described alkyl group which may have a substituent is usually 1 to 10.

$R^{d3}$ represents $COO^-M_d^+$ or $SO_3^-M_d^+$. $M_d^+$ represents a cation.

Examples of the above-described cation include known cations, and specific examples thereof include a monovalent cation such as $H^+$ (proton), a monovalent organic cation, and a monovalent inorganic cation, and $K^+$ or $Na^+$ is preferable.

nd1 and nd2 each independently represent an integer of 0 to 4.

nd1 and nd2 may be the same or different from each other, and preferably represent the same integer.

Hereinafter, the compound represented by Formula (V) will be described in detail.

$R^{e1}$ and $R^{e2}$ each independently represent an alkyl group which may have a substituent.

Examples of $R^{e1}$ and $R^{e2}$ include the alkyl group which may have a substituent, represented by $R^{d1}$ and $R^{d2}$

described above.

$R^{e3}$ represents $COO^-M_e^+$ or $SO_3^-M_e^+$. $M_e^+$ represents a cation.

$M_e^+$ is the same as $M_d^+$, and a suitable aspect thereof is also the same.

ne represents 0 or 1.

Examples of the leuco coloring agent which forms color by the action of acid (acid color-forming leuco coloring agent) include 3,3-bis(2-methyl-1-octyl-3-indolyl)phthalide, 6'-(dibutylamino)-2'-bromo-3'-methylspiro[phthalido-3,9'-xanthene], 3-(4-diethylamino-2-ethoxyphenyl)-3-(1-ethyl-2-methylindol-3-yl)-4-azaphthalide, 3-(4-diethylamino-2-ethoxyphenyl)-3-(1-n-octyl-2-methylindol-3-yl)phthalide, 3-[2,2-bis(1-ethyl-2-methylindol-3-yl)vinyl]-3-(4-diethylaminophenyl)-phthalide, 2-anilino-6-dibutylamino-3-methylfluorane, 6-diethylamino-3-methyl-2-(2,6-xylidino)-fluorane, 2-(2-chloroanilino)-6-dibutylaminofluorane, 3,3-bis(4-dimethylaminophenyl)-6 -dimethylaminophthalide, 2-anilino-6-diethylamino-3-methylfluorane, 9-[ethyl(3-methylbutyl)amino]spiro[12H-benzo[a]xanthene-12,1'(3'H)isobenzofuran]-3'-one, 2'-methyl-6'-(N-p-tolyl-N-ethylamino)spiro[isobenzofuran-1(3H),9'-[9H]xanthene]-3-one, 3',6'-bis(diethylamino)-2-(4-nitrophenyl)spiro[isoindole-1,9'-xanthene]-3-one, 9-(N-ethyl-N-isopentylamino)spiro[benzo[a]xanthene-12,3'-phthalide], 2'-anilino-6'-(N-ethyl-N-isopentylamino)-3'-methylspiro[phthalide-3,9'-[9H]xanthene], and 6'-(diethylamino)-1',3'-dimethylfluorane.

From the viewpoint that the effect of the present invention is more excellent, the color-forming agent is preferably a compound having any of an indolylphthalide structure or an azaindolylphthalide structure, and preferably a compound having an indolylphthalide structure.

The compound having an indolylphthalide structure is a compound having an indolylphthalide structure as a partial structure. As described above, the above-described compound having an indolylphthalide structure (indolylphthalide-based compound) and the above-described compound having an azaindolylphthalide structure (azaindolylphthalide-based compound) function as the color-forming agent. That is, the above-described compounds correspond to a color-forming agent having an indolylphthalide structure (particularly, an acid color forming agent) and a color-forming agent having an azaindolylphthalide structure.

The number of indolylphthalide structures in the compound having an indolylphthalide structure is not particularly limited, and may be 1 or a plural number. Among these, from the viewpoint that the effect of the present invention is more excellent, 2 or more is preferable, and 2 is more preferable.

As the compound having an indolylphthalide structure, a compound represented by General Formula (A) or a compound represented by General Formula (B) is preferable, and a compound represented by General Formula (B) is more preferable.

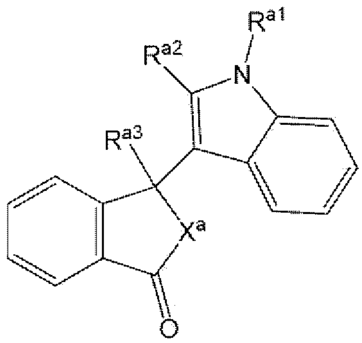

(A)

In General Formula (A), $R^{a1}$ and $R^{a2}$ each independently represent a hydrogen atom or an alkyl group which may have a substituent.

The number of carbon atoms in the alkyl group represented by $R^{a1}$ is not particularly limited, but from the viewpoint that the effect of the present invention is more excellent, 1 to 30 is preferable, 1 to 20 is more preferable, 1 to 12 is still more preferable, and 5 to 10 is particularly preferable.

The number of carbon atoms in the alkyl group represented by $R^{a2}$ is not particularly limited, but from the viewpoint that the effect of the present invention is more excellent, 1 to 10 is preferable, 1 to 5 is more preferable, and 1 to 3 is still more preferable.

Among these, from the viewpoint that the effect of the present invention is more excellent, $R^{a1}$ and $R^{a2}$ preferably represent an alkyl group which may have a substituent, and more preferably represent an unsubstituted alkyl group.

$R^{a3}$ represents a hydrogen atom, an alkyl group which may have a substituent, or an aryl group which may have a substituent.

The number of carbon atoms in the alkyl group represented by $R^{a3}$ is not particularly limited, but from the viewpoint that the effect of the present invention is more excellent, 1 to 10 is preferable and 1 to 5 is more preferable.

The aryl group represented by $R^{a3}$ may have a monocyclic structure or a polycyclic structure.

Among these, as $R^{a3}$, from the viewpoint that the effect of the present invention is more excellent, an aryl group which may have a substituent is preferable, and an aryl group which has a substituent is more preferable.

$X^a$ represents -O- or -$NR^{a4}$-.

Among these, from the viewpoint that the effect of the present invention is more excellent, $X^a$ is preferably -O-.

$R^{a4}$ represents a hydrogen atom, an alkyl group which may have a substituent, or an aryl group which may have a substituent.

The number of carbon atoms in the alkyl group represented by $R^{a4}$ is not particularly limited, but from the viewpoint that the effect of the present invention is more excellent, 1 to 10 is preferable and 1 to 5 is more preferable.

The aryl group represented by $R^{a4}$ may have a monocyclic structure or a polycyclic structure.

A molecular weight of the compound represented by General Formula (A) is not particularly limited, but is preferably 300 or more and more preferably 500 or more. The upper limit thereof is not particularly limited, but is preferably 2,000 or less and more preferably 1,000 or less.

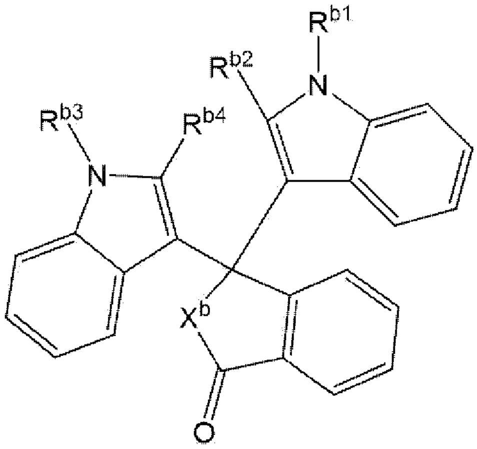

(B)

In General Formula (B), $R^{b1}$ to $R^{b4}$ each independently represent a hydrogen atom or an alkyl group which may have a substituent.

The number of carbon atoms in the alkyl group represented by $R^{b1}$ and $R^{b3}$ is not particularly limited, but from the viewpoint that the effect of the present invention is more excellent, 1 to 30 is preferable, 1 to 20 is more preferable, 1 to 12 is still more preferable, and 5 to 10 is particularly preferable.

The number of carbon atoms in the alkyl group represented by $R^{b2}$ and $R^{b4}$ is not particularly limited, but from the viewpoint that the effect of the present invention is more excellent, 1 to 10 is preferable, 1 to 5 is more preferable, and 1 to 3 is still more preferable.

Among these, from the viewpoint that the effect of the present invention is more excellent, $R^{b1}$ to $R^{b4}$ are preferably an alkyl group which may have a substituent, and more preferably an unsubstituted alkyl group.

$X^b$ represents -O- or -$NR^{b5}$-.

Among these, from the viewpoint that the effect of the present invention is more excellent, $X^b$ is preferably -O-.

$R^{b5}$ represents a hydrogen atom, an alkyl group which may have a substituent, or an aryl group which may have a substituent.

The number of carbon atoms in the alkyl group represented by $R^{b5}$ is not particularly limited, but from the viewpoint that the effect of the present invention is more excellent, 1 to 10 is preferable and 1 to 5 is more preferable.

The aryl group represented by $R^{b5}$ may have a monocyclic structure or a polycyclic structure.

A molecular weight of the compound represented by General Formula (B) is not particularly limited, but is preferably 300 or more and more preferably 500 or more. The upper limit thereof is not particularly limited, but is preferably 2,000 or less and more preferably 1,000 or less.

A content of the color-forming agent in the ultraviolet-sensing layer is not particularly limited, but from the viewpoint that the effect of the present invention is more excellent, the content thereof per unit area ($m^2$) of the ultraviolet-sensing layer 0.500 g/$m^2$ or less, 0.300 g/$m^2$ or less, still more preferably 0.140 g/$m^2$ or less, and particularly preferably 0.070 g/$m^2$ or less. The lower limit thereof is not particularly limited, but is preferably 0.020 g/$m^2$ or more and more preferably 0.030 g/$m^2$ or more. By setting the content of the color-forming agent in the ultraviolet-sensing layer within the above-described range, since excessive absorption of 222 nm by the color-forming agent is suppressed, the minimum amount of color-forming agent required for the color-forming reaction can be used, and as a result, it is presumed that the difference in optical density can be set to 0.20 or more.

The above-described content of the color-forming agent can be calculated by cutting out an area having many

ultraviolet-sensing layers from the ultraviolet-sensing member, immersing the ultraviolet-sensing layers in methanol for 2 days, and then analyzing the obtained solvent by liquid chromatography. The methanol is prevented from volatilizing during the immersion. As necessary, a calibration curve of the content of the color-forming agent to be detected may be created before the measurement of the liquid chromatography.

Measurement conditions of the liquid chromatography are as follows.

Equipment: Nexera manufactured by Shimadzu Corporation
Column: Capcell pak C18 UG-120
Eluent: water/methanol
Oven: 40°C
Injection: 5 μL
Detection: maximal absorption wavelength of color-forming agent to be detected
Flow Rate: 0.2 mL/min

(Photoactivator)

The ultraviolet-sensing layer preferably contains a photoactivator.

The photoactivator is not particularly limited as long as it is a compound which is activated by light, but the photoactivator activated by light preferably acts on the color-forming agent to form color, and is preferably a compound activated by ultraviolet rays. The photoactivator is preferably any one or more of a photooxidant or a photoacid generator. In a case where the ultraviolet-sensing member contains the color-forming agent which forms color by being oxidized, the photoactivator preferably includes a photooxidant, and in a case where the ultraviolet-sensing member contains the color-forming agent which forms color by action of acid, the photoactivator preferably includes a photoacid generator.

The photoactivator may be used alone or in combination of two or more kinds thereof.

From the viewpoint that the effect of the present invention is more excellent, a mass ratio of a content of the photoactivator to a content of the color-forming agent (photoactivator/color-forming agent (mass ratio)) is preferably more than 1.00, more preferably 3.00 or more, still more preferably 8.00 or more, and particularly preferably 10.00 or more. The upper limit thereof is not particularly limited, but is preferably 40.00 or less, more preferably 30.00 or less, still more preferably 25.00 or less, and particularly preferably 20.00 or less. By setting the mass ratio of the content of the photoactivator to the content of the color-forming agent within the above-described range, since excessive absorption of 222 nm by the color-forming agent is suppressed, the photoactivator efficiently absorbs 222 nm, and as a result, it is presumed that the color-forming reaction proceeds efficiently and the difference in optical density can be set to 0.20 or more.

The above-described mass ratio of the content of the photoactivator to the content of the color-forming agent can be measured by liquid chromatography after methanol extraction in the same manner as the above-described content of the color-forming agent. The photoactivator is detected at the maximal absorption wavelength of the photoactivator to be detected, the color-forming agent is detected at the maximal absorption wavelength of the color-forming agent to be detected, and the mass ratio thereof is obtained.

· Photooxidant

The photooxidant is preferably a compound which can cause the forming of the color-forming agent by being activated by ultraviolet rays to generate a radical and exhibit an action of extracting the hydrogen atom of the color-forming agent.

Among these, the photooxidant is preferably one or more of a radical generator and an organic halogen compound. It is also preferable to use the radical generator and the organic halogen compound in combination as the photoacid generator. In a case where the radical generator and the organic halogen compound are used in combination, from the viewpoint that the gradation properties of the colored part are more excellent, a content ratio (radical generator/organic halogen compound (mass ratio)) of the radical generator to the organic halogen compound is preferably 0.1 to 10 and more preferably 0.5 to 5.

·· Radical generator

The radical generator is not particularly limited as long as it is a compound which is activated by ultraviolet rays to generate a radical.

As the radical generator, a hydrogen-extracting radical generator is preferable. The hydrogen-extracting radical generator exhibits an action of extracting hydrogen atoms from the color-forming agent to promote the oxidation of the color-forming agent.

Examples of the radical generator include azide polymers described in The Lecture Summary, p. 55 for the Spring Meeting of the Society of Photographic Science and Technology of Japan, 1968; azide compounds described in US3282693A, such as 2-azidobenzoxazole, benzoylazide, and 2-azidobenzimidazole; compounds described in US3615568A, such as 3'-ethyl-1-methoxy-2-pyridothiacyanine perchlorate, 1-methoxy-2-methylpyridinium, and p-toluenesulfonate; lophine dimer compounds described in JP1987-039728B (JP-S62-039728B), such as a 2,4,5-triarylimidazole dimer; benzophenone; p-aminophenyl ketone; polynuclear quinone; and thioxanthenone.
Among these, one or more selected from a lophine dimer and benzophenone is preferable, and a lophine dimer is more preferable.
Examples of the lophine dimer include a hexaarylbiimidazole compound. As the hexaarylbiimidazole-based compound, compounds described in paragraph 0047 of WO2016/017701A can be referred to, the contents of which are incorporated in the present specification.
Among these, 2,2'-bis(2-chlorophenyl)-4,4',5,5'-tetraphenyl-1,2'-biimidazole is preferable. As the 2,2'-bis(2-chlorophenyl)-4,4',5,5'-tetraphenyl-1,2'-biimidazole, for example, "B-IMD" (manufactured by KUROGANE KASEI Co., Ltd.) and "B-CIM" (manufactured by Hodogaya Chemical Co., Ltd.) can be used.
As the lophine dimer, a compound represented by General Formula (1) is also preferable.

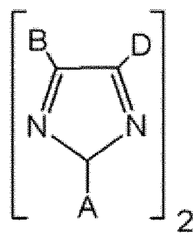

(1)

In the formula, A, B, and D each independently represent a carbocyclic or heteroaryl group, which is unsubstituted or substituted with a substituent which does not interfere with dissociation of the dimer to an imidazolyl group or the oxidation of the color-forming agent.
It is preferable that B and D are each independently unsubstituted or have 1 to 3 substituents, and it is preferable that A is unsubstituted or has 1 to 4 substituents.
As the compound represented by General Formula (1) and a method of preparing the compound, a finding known as the lophine dimer or the like can be utilized. For example, the description of column 4, line 22 and column 6, line 3 of US3552973A can be referred to, the contents of which are incorporated in the present specification.
The radical generator may be used alone or in combination of two or more kinds thereof.

·· Organic halogen compound

The organic halogen compound can promote the oxidation of the color-forming agent.
From the viewpoint that the gradation properties of the colored part are more excellent, the organic halogen compound is preferably a compound in which the number of halogen atoms in the molecule is 3 or more. The upper limit value of the number of halogen atoms is preferably 9 or less. The organic halogen compound is a compound other than the lophine dimer and the benzophenone.
The organic halogen compound may be used alone or in combination of two or more kinds thereof.
Examples of the organic halogen compound include compounds represented by General Formulae (2) to (7).

$$P^0\text{-}CX_3 \qquad (2)$$

In the formula, $P^0$ represents a hydrogen atom, a halogen atom, an alkyl group which may have a substituent, or an aryl group which may have a substituent. X's each independently represent a halogen atom.
Examples of the halogen atom represented by $P^0$ and X include a fluorine atom, a chlorine atom, a bromine atom, and an iodine atom, and a chlorine atom or a bromine atom is preferable.
Examples of the substituent which can be included in the alkyl group and aryl group represented by $P^0$ include a hydroxy group, a halogen atom, an alkyl group having 1 to 6 carbon atoms, a haloalkyl group having 1 to 6 carbon atoms, an acetyl group, and an alkoxy group having 1 to 6 carbon atoms.
Examples of the compound represented by General Formula (2) include trichloromethane, tribromomethane, carbon tetrachloride, carbon tetrabromide, p-nitrobenzotribromide, bromotrichloromethane, pensitrichloride, hexabromoethane, iodoform, 1,1,1-tribromo-2-methyl-2-propanol, 1,1,2,2-tetrabromoethane, 2,2,2-tribromoethanol, and 1,1,1-trichloro-2-methyl-2-propanol.

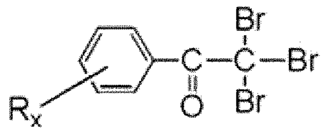

(3)

In the formula, R represents a substituent. x represents an integer of 0 to 5.

Examples of the substituent represented by R include a nitro group, a halogen atom, an alkyl group having 1 to 3 carbon atoms, a haloalkyl group having 1 to 3 carbon atoms, an acetyl group, a haloacetyl group, and an alkoxy group having 1 to 3 carbon atoms.

In a case where a plurality of R's are present in the formula, the R's may be the same or different from each other.

x is preferably 0 to 3.

Examples of the compound represented by General Formula (3) include o-nitro-α,α,α-tribromoacetophenone, m-nitro-α,α,α-tribromoacetophenone, p-nitro-α,α,α-tribromoacetophenone, α,α,α-tribromoacetophenone, and α,α,α-tribromo-3,4-cycloacetophenone.

$R^1$-$SO_2$-$X^1$ (4)

In the formula, $R^1$ represents an alkyl group which may have a substituent or an aryl group which may have a substituent. $X^1$ represents a halogen atom.

As the alkyl group represented by $R^1$, an alkyl group having 1 to 20 carbon atoms is preferable, an alkyl group having 1 to 10 carbon atoms is more preferable, and an alkyl group having 1 to 6 carbon atoms is still more preferable.

As the aryl group represented by $R^1$, an aryl group having 6 to 20 carbon atoms is preferable, an aryl group having 6 to 14 carbon atoms is more preferable, and an aryl group having 6 to 10 carbon atoms is still more preferable.

Examples of the substituent which can be included in the alkyl group and aryl group represented by $R^1$ include a nitro group, a halogen atom, an alkyl group having 1 to 3 carbon atoms, a haloalkyl group having 1 to 3 carbon atoms, an acetyl group, a haloacetyl group, and an alkoxy group having 1 to 3 carbon atoms.

Examples of the halogen atom represented by $X^1$ include a fluorine atom, a chlorine atom, a bromine atom, and an iodine atom, and a chlorine atom, a bromine atom, or an iodine atom is preferable and a chlorine atom or a bromine atom is more preferable.

Examples of the compound represented by General Formula (4) include 2,4-dinitrobenzenesulfonyl chloride, o-nitrobenzenesulfonyl chloride, m-nitrobenzenesulfonyl chloride, 3,3'-diphenylsulfonedisulfonyl chloride, ethanesulfonyl chloride, p-bromobenzenesulfonyl chloride, p-nitrobenzenesulfonyl chloride, p-3-benzenesulfonyl chloride, p-acetamidobenzenesulfonyl chloride, p-chlorobenzenesulfonyl chloride, p-toluenesulfonyl chloride, methanesulfonyl chloride, and henzenesulfonyl bromide.

$R^2$-S-$X^2$ (5)

In the formula, $R^2$ represents an alkyl group which may have a substituent or an aryl group which may have a substituent. $X^2$ represents a halogen atom.

The alkyl group which may have a substituent and the aryl group which may have a substituent, represented by $R^2$, are the same as those of $R^1$ in General Formula (4), and suitable aspects thereof are also the same.

Examples of the halogen atom represented by $X^2$ include a fluorine atom, a chlorine atom, a bromine atom, and an iodine atom, and a chlorine atom, a bromine atom, or an iodine atom is preferable and a chlorine atom or a bromine atom is more preferable.

Examples of the compound represented by General Formula (5) include 2,4-dinitrobenzenesulfenyl chloride and o-nitrobenzenesulfenyl chloride.

$R^3$-$L^1$-$CX^3X^4X^5$ (6)

In the formula, $R^3$ represents an aryl group which may have a substituent or a heteroaryl group which may have a substituent. $L^1$ represents -SO- or $SO_2$-. $X^3$, $X^4$, and $X^5$ each independently represent a hydrogen atom or a halogen atom. However, all of $X^3$, $X^4$, and $X^5$ are not hydrogen atoms at the same time.

As the aryl group represented by $R^3$, an aryl group having 6 to 20 carbon atoms is preferable, an aryl group having 6 to 14 carbon atoms is more preferable, and an aryl group having 6 to 10 carbon atoms is still more preferable.
As the heteroaryl group represented by $R^3$, a heteroaryl group having 4 to 20 carbon atoms is preferable, a heteroaryl group having 4 to 13 carbon atoms is more preferable, and a heteroaryl group having 4 to 9 carbon atoms is still more preferable.
Examples of the substituent which can be included in the aryl group and heteroaryl group represented by $R^3$ include a nitro group, a halogen atom, an alkyl group having 1 to 3 carbon atoms, a haloalkyl group having 1 to 3 carbon atoms, an acetyl group, a haloacetyl group, and an alkoxy group having 1 to 3 carbon atoms.
Examples of the halogen atom represented by $X^3$, $X^4$, and $X^5$ include a fluorine atom, a chlorine atom, a bromine atom, and an iodine atom, and a chlorine atom, a bromine atom, or an iodine atom is preferable and a chlorine atom or a bromine atom is more preferable.
Examples of the compound represented by General Formula (6) include hexabromodimethyl sulfoxide, pentabromodimethyl sulfoxide, hexabromodimethylsulfone, trichloromethylphenylsulfone, tribromomethylphenylsulfone (BMPS), trichloro-p-chlorophenylsulfone, tribromomethyl-p-nitrophenylsulfone, 2-trichloromethylbenzothiazolesulfone, 4,6-simethylbyrimidine-2-tribromomethylsulfone, tetrabromodimethylsulfone, 2,4-dichlorophenyl-trichloromethylsulfone, 2-methyl-4-chlorophenyltri chloromethylsulfone, 2,5-dimethyl-4-chlorophenyltrichloromethylsulfone, 2,4-dichlorophenyltrimethylsulfone, and tri-p-tolylsulfonium trifluoromethanesulfonate. Among these, trichloromethylphenylsulfone or tribromomethylphenylsulfone (BMPS) is preferable.

$$R^4CX^6X^7X^8 \qquad (7)$$

In the formula, $R^4$ represents a heteroaryl group which may have a substituent. $X^6$, $X^7$, and $X^8$ each independently represent a hydrogen atom or a halogen atom. However, all of $X^6$, $X^7$, and $X^8$ are not hydrogen atoms at the same time.
As the heteroaryl group represented by $R^4$, a heteroaryl group having 4 to 20 carbon atoms is preferable, a heteroaryl group having 4 to 13 carbon atoms is more preferable, and a heteroaryl group having 4 to 9 carbon atoms is still more preferable.
Examples of the substituent which can be included in the heteroaryl group represented by $R^4$ include a nitro group, a halogen atom, an alkyl group having 1 to 3 carbon atoms, a haloalkyl group having 1 to 3 carbon atoms, an acetyl group, a haloacetyl group, and an alkoxy group having 1 to 3 carbon atoms.
Examples of the halogen atom represented by $X^6$, $X^7$, and $X^8$ include a fluorine atom, a chlorine atom, a bromine atom, and an iodine atom, and a chlorine atom, a bromine atom, or an iodine atom is preferable and a chlorine atom or a bromine atom is more preferable.
Examples of the compound represented by General Formula (7) include tribromoquinaldine, 2-tribromomethyl-4-methylquinoline, 4-tribromomethylpyrimidine, 4-phenyl-6-tribromomethylpyrimidine, 2-trichloromethyl-6-nitrobenzothiazole, 1-phenyl-3-trichloromethylpyrazole, 2,5-ditribromomethyl-3,4-dibromothiophene, 2-trichloromethyl-3-(p-butoxystyryl)-1,3,4-oxadiazole, 2,6-didolychloromethyl-4-(p-methoxyphenyl)-triazine, and 2-(4-methylphenyl)-4,6-bis(trichloromethyl)-1,3,5-triazine.
Among these, as the organic halogen compound, the compound represented by General Formula (3), the compound represented by General Formula (6), or the compound represented by General Formula (7) is preferable, and from the viewpoint that the effect of the present invention is more excellent, the compound represented by General Formula (6) is more preferable. The reason why the effect of the present invention is more excellent is not clear, but it is presumed that the above-described compound represented by General Formula (6) has good compatibility with the wavelength of 222 nm.
As the halogen atom included in the above-described compound, a chlorine atom, a bromine atom, or an iodine atom is preferable, and a chlorine atom or a bromine atom is more preferable.
The organic halogen compound may be used alone or in combination of two or more kinds thereof.

· Photoacid generator

The photoacid generator is preferably a compound which is cleaved by ultraviolet rays to generate an acid and can cause the forming of the color-forming agent by the action of acid.
Examples of the photoacid generator include a non-ionic photoacid generator and an ionic photoacid generator, and from the viewpoint that the effect of the present invention is more excellent, a non-ionic photoacid generator is preferable. Examples of the non-ionic photoacid generator include an organic halogen compound and an oxime compound, and among these, from the viewpoint that the effect of the present invention is more excellent, an organic halogen compound is preferable, and the compound represented by General Formula (6) described above is more preferable.
From the viewpoint that the gradation properties of the colored part are more excellent, the organic halogen compound is preferably a compound in which the number of halogen atoms in the molecule is 3 or more. The upper

limit value of the number of halogen atoms is preferably 9 or less.

The organic halogen compound may be used alone or in combination of two or more kinds thereof.

Specific examples of the organic halogen compound include the same organic halogen compounds as those mentioned as the photooxidant in the upper part.

Examples of the ionic photoacid generator include a diazonium salt, an iodonium salt, and a sulfonium salt, and an iodonium salt or a sulfonium salt is preferable. Examples of the ionic photoacid generator developing agent include compounds described in JP1987-161860A (JP-S62-161860A), JP1986-67034A (JP-S61-67034A), and JP1987-050382A (JP-S62-050382A), the contents of which are incorporated in the present specification.

In addition, the photoacid generator is not particularly limited as long as it is a compound which generates an acid by light, and the photoacid generator may be a photoacid generator which generates an inorganic acid such as a hydrogen halide (for example, hydrochloric acid), a sulfuric acid, and a nitric acid, or may be a photoacid generator which generates an organic acid such as a carboxylic acid and a sulfonic acid. From the viewpoint that the effect of the present invention is more excellent, a photoacid generator which generates an inorganic acid is preferable, and a photoacid generator which generates a hydrogen halide is more preferable.

Specific examples of the photoacid generator include triarylsulfonium hexafluorophosphate, triarylsulfonium arsenate and triarylsulfonium antimonate, diaryliodonium hexafluorophosphate, diaryliodonium arsenate and diaryliodonium antimonate, dialkylphenacylsulfonium tetrafluoroborate and dialkylphenacylsulfonium hexafluorophosphate, dialkyl-4-hydroxyphenylsulfonium tetrafluoroborate and dialkyl-4-hydroxyphenylsulfonium hexafluorophosphate, N-promosuccinimide, tribromomethylphenylsulfone, diphenyliodine, 2-trichloromethyl-5-(p-butoxystyryl)-1.3.4-oxadiazole, and 2.6-ditrichloromethyl-4-(p-methoxyphenyl)-triazine.

The ultraviolet-sensing layer may contain a component other than the color-forming agent and the photoactivator.

Examples of other components include other components described in the first embodiment described later (polymer binder, reducing agent, light stabilizer, crosslinking agent, sensitizer, ultraviolet absorber, surfactant, colorant, and the like).

Hereinafter, the ultraviolet-sensing member will be described in detail with reference to specific aspects.

[First embodiment]

The ultraviolet-sensing member according to the first embodiment of the present invention is an ultraviolet-sensing member including an ultraviolet-sensing layer containing a microcapsule which contains a photoactivator, a color-forming agent, and a solvent having a heteroatom. That is, the above-described ultraviolet-sensing members according to the embodiments A to C of the present invention may be an ultraviolet-sensing member including an ultraviolet-sensing layer containing a microcapsule which contains a photoactivator, a color-forming agent, and a solvent having a heteroatom.

Hereinafter, the solvent having a heteroatom is also referred to as "specific solvent".

In the first embodiment, any one of the difference between C1 and C2, the difference between Y1 and Y2, or the difference between M1 and M2 described above can be easily controlled to 0.20 or more.

Fig. 1 is a schematic cross-sectional view of an example of the first embodiment of the ultraviolet-sensing member.

An ultraviolet-sensing member 10 includes a support 12 and an ultraviolet-sensing layer 14 which is disposed on one surface of the support 12 and contains a microcapsule containing a photoactivator, a color-forming agent, and a specific solvent. In the ultraviolet-sensing layer 14 which is irradiated with ultraviolet rays, a colored part (not shown) which forms color with a color optical density corresponding to an amount of ultraviolet irradiation is formed.

As described above, although Fig. 1 shows an aspect in which the ultraviolet-sensing member is a sheet-like shape, the present invention is not limited to this aspect.

As will be described later, it is sufficient that the ultraviolet-sensing member 10 includes the ultraviolet-sensing layer 14, and the support 12 may not be included.

Furthermore, the ultraviolet-sensing member 10 shown in Fig. 1 has a two-layer configuration of the support 12 and the ultraviolet-sensing layer 14, but the present invention is not limited to this aspect. As will be described later, the ultraviolet-sensing member 10 may include a layer other than the support 12 and the ultraviolet-sensing layer 14 (for example, a reflective layer, a glossy layer, a filter layer, and the like).

In a case where the ultraviolet-sensing layer included in the ultraviolet-sensing member is irradiated with ultraviolet rays for measuring an amount of ultraviolet irradiation, in a region irradiated with ultraviolet rays (ultraviolet-irradiated region), a colored part (color-formed image) is formed with a color optical density corresponding to the amount of ultraviolet irradiation (for example, integrated illuminance). The fact that color is formed with the color optical density corresponding to the amount of ultraviolet irradiation means that the color-formed image has gradation properties according to the amount of ultraviolet irradiation.

The above-described main color-forming mechanism of the ultraviolet-sensing layer is derived from the microcapsule contained in the ultraviolet-sensing layer. In a case where the ultraviolet-sensing layer is irradiated with ultraviolet rays, a color-forming agent present in the ultraviolet-irradiated region usually forms color in the microcapsule. Specifically, the photoactivator absorbs ultraviolet rays and is activated to generate an acid and/or a radical, and the color-forming agent forms color by reaction with this acid and/or radical. In this case, an amount of acid and/or radical generated from the photoactivator varies depending on the irradiated amount of ultraviolet irradiation, and an amount of the color-forming agent which forms color also varies depending on the amount of acid and/or radical generated from the photoactivator. As a result, in the ultraviolet-irradiated region of the ultraviolet-sensing layer, shade of the color optical density is generated according to the irradiated amount of ultraviolet irradiation, and the colored part is formed with the color optical density corresponding to the amount of ultraviolet irradiation.

The present inventors have presumed that, in a case where the microcapsule in the ultraviolet-sensing layer contains the specific solvent, since the specific solvent has little absorption at a wavelength of 222 nm, and the specific solvent has high solubility of the photoactivator and the color-forming agent, it is difficult to inhibit the coloring reaction, thereby contributing to improved sensitivity at the wavelength of 222 nm.

A lower limit value of a thickness of the ultraviolet-sensing member 10 is preferably 5 μm or more and more preferably 25 μm or more. In addition, the upper limit value thereof is preferably 1 cm or less and more preferably 2 mm or less.

Hereinafter, each member of the first embodiment of the ultraviolet-sensing member will be described in detail.

<<Support>>

The support is a member for supporting the ultraviolet-sensing layer.

In a case where the ultraviolet-sensing layer itself can be handled, the ultraviolet-sensing member may not include the support.

Examples of the support include a resin sheet, paper (including synthetic paper), cloth (including woven fabric and nonwoven fabric), glass, wood, and metal. As the support, a resin sheet or paper is preferable, a resin sheet or synthetic paper is more preferable, and a resin sheet is still more preferable.

Examples of a material of the resin sheet include a polyethylene-based resin, a polypropylene-based resin, a cyclic polyolefin-based resin, a polystyrene-based resin, an acrylonitrile-styrene copolymer, an acrylonitrile-butadiene-styrene copolymer, a polyvinyl chloride-based resin, a fluorine-based resin, a poly(meth)acrylic resin, a polycarbonate-based resin, a polyester-based resin (polyethylene terephthalate, polyethylene naphthalate, and the like), a polyamide-based resin such as various nylons, a polyimide-based resin, a polyamide-imide-based resin, a polyaryl phthalate-based resin, a silicone-based resin, a polysulfone-based resin, a polyphenylene sulfide-based resin, a polyethersulfone-based resin, a polyurethane-based resin, an acetal-based resin, and a cellulose-based resin.

Examples of the synthetic paper include paper in which many microvoids are formed by biaxially stretching polypropylene or polyethylene terephthalate (YUPO and the like); paper produced from synthetic fibers such as polyethylene, polypropylene, polyethylene terephthalate, and polyamide; and paper in which these papers are laminated on part, one side, or both sides thereof.

In addition, examples of another suitable aspect of the resin sheet include a white resin sheet formed by dispersing a white pigment in a resin. Examples of a material of the resin in the above-described white resin sheet include the same materials as those in the resin sheet described above.

The white resin sheet has ultraviolet reflectivity. Therefore, in a case where the support is the white resin sheet, since ultraviolet rays irradiated to the ultraviolet-sensing member are reflected by the support, it is possible to suppress scattering of the ultraviolet rays inside the ultraviolet-sensing member. As a result, accuracy of detecting the amount of ultraviolet irradiation in the ultraviolet-sensing member can be further improved.

As the white pigment, white pigments described in paragraph 0080 of WO2016/017701A can be referred to, the contents of which are incorporated in the present specification.

As the white resin sheet, for example, a white polyester sheet is preferable, and a white polyethylene terephthalate sheet is more preferable.

Examples of a commercially available product of the white resin sheet include YUPO (manufactured by YUPO Corporation), LUMIRROR (manufactured by Toray Industries Inc.), and CRISPER (manufactured by Toyobo Co., Ltd.).

A lower limit value of a thickness of the support is preferably 5 μm or more, more preferably 25 μm or more, and still more preferably 50 μm or more. In addition, the upper limit value thereof is preferably 1 cm or less, more preferably 2 mm or less, and still more preferably 500 μm or less.

«Ultraviolet-sensing layer»

The ultraviolet-sensing layer contains a microcapsule (hereinafter, also referred to as "specific microcapsule

A") containing a photoactivator, a color-forming agent, and a specific solvent.

Hereinafter, various components which can be contained in the ultraviolet-sensing layer will be described in detail.

<Specific microcapsule A>

The ultraviolet-sensing layer contains the specific microcapsule A.

Hereinafter, first, materials constituting the specific microcapsule A will be described in detail.

The specific microcapsule A usually includes a core portion and a capsule wall for encompassing a core material (encompassed substance (hereinafter, also referred to as an encompassed component)) forming the core portion.

The specific microcapsule A contains, as the core material (encompassed component), the photoactivator, the color-forming agent, and the specific solvent.

Examples of one suitable aspect of the specific microcapsule A include an aspect in which the photoactivator is the photooxidant and the color-forming agent is the color-forming agent which forms color by being oxidized.

In addition, examples of another suitable aspect of the specific microcapsule A include an aspect in which the photoactivator is the photoacid generator and the color-forming agent is the color-forming agent which forms color by action of acid.

It is preferable that the specific microcapsule A prevents contact between substances inside and outside the capsule by a substance-separating action of the capsule wall at normal temperature. Specific examples thereof include JP1984-190886A (JP-S59-190886A) and JP1985-242094A (JP-S60-242094A), the contents of which are incorporated in the present specification.

(Capsule wall)

It is preferable that the capsule wall of the specific microcapsule A is substantially composed of a resin. The term "substantially composed of a resin" means that a content of the resin with respect to the total mass of the capsule wall is 90% by mass or more, preferably 100% by mass. That is, it is preferable that the capsule wall of the specific microcapsule is composed of a resin.

Examples of the above-described resin include polyurethane, polyurea, polyester, polycarbonate, a urea-formaldehyde resin, a melamine-formaldehyde resin, polystyrene, a styrene-methacrylate copolymer, gelatin, polyvinylpyrrolidone, and polyvinyl alcohol. Among these, from the viewpoint that the sensitivity at the wavelength of 222 nm can be further improved by forming a dense crosslinking structure which prevents encompassed substances from leaking and by controlling the transmittance at the wavelength of 222 nm, one or more selected from the group consisting of polyurea, polyurethane urea, and polyurethane are more preferable.

The polyurea is a polymer having a plurality of urea bonds, and is preferably a reaction product formed from a raw material containing polyamine and polyisocyanate.

It is also possible to synthesize the polyurea using the polyisocyanate without using the polyamine, by utilizing the fact that a part of the polyisocyanate reacts with water to form the polyamine.

In addition, the polyurethane urea is a polymer having a urethane bond and a urea bond, and is preferably a reaction product formed from a raw material containing polyol, polyamine, and polyisocyanate.

In a case where the polyol is reacted with the polyisocyanate, a part of the polyisocyanate reacts with water to form the polyamine, and as a result, the polyurethane urea is obtained.

In addition, the polyurethane is a polymer having a plurality of urethane bonds, and is preferably a reaction product formed from a raw material containing polyol and polyisocyanate.

The polyisocyanate preferably has an aromatic ring or an alicyclic ring.

Among these, from the viewpoint that the effect of the present invention is more excellent, the polyisocyanate preferably has an alicyclic ring. In a case of using the polyisocyanate having an alicyclic ring, the transparency of the microcapsule wall is excellent, so that the sensitivity at the wavelength of 222 nm is more excellent.

Examples of the above-described aromatic ring include an aromatic hydrocarbon ring and an aromatic heterocyclic ring, and an aromatic hydrocarbon ring is preferably used.

The above-described aromatic hydrocarbon ring may have a substituent.

The number of carbon atoms in the above-described aromatic hydrocarbon ring is not particularly limited, but is preferably 6 to 30, more preferably 6 to 18, and still more preferably 6 to 10.

Examples of the aromatic hydrocarbon ring include a benzene ring.

The number of aromatic rings in the polyisocyanate is not particularly limited, may be 1 or 2 or more, and is preferably 1.

The above-described alicyclic ring may have a substituent.

The number of carbon atoms in the above-described alicyclic ring is not particularly limited, but is preferably 3

to 30, more preferably 3 to 18, and still more preferably 6 to 10.

Examples of the alicyclic ring include a cyclohexane ring.

The number of alicyclic rings in the polyisocyanate is not particularly limited, may be 1 or 2 or more, and is preferably 1 to 3.

Examples of the aromatic polyisocyanate include an aromatic diisocyanate, and examples thereof include m-phenylene diisocyanate, p-phenylene diisocyanate, 2,6-tolylene diisocyanate, 2,4-tolylene diisocyanate, naphthalene-1,4-diisocyanate, diphenylmethane-4,4'-diisocyanate, 3,3'-dimethoxy-biphenyl diisocyanate, 3,3'-dimethyldiphenylmethane-4,4'-diisocyanate, xylylene-1,4-diisocyanate, xylylene-1,3-diisocyanate, 4-chloroxylylene-1,3-diisocyanate, 2-methylxylylene-1,3-diisocyanate, 4,4'-diphenylpropane diisocyanate, and 4,4'-diphenylhexafluoropropane diisocyanate.

Examples of the aliphatic polyisocyanate include an aliphatic diisocyanate, and examples thereof include trimethylene diisocyanate, hexamethylene diisocyanate, propylene-1,2-diisocyanate, butylene-1,2-diisocyanate, cyclohexylene-1,2-diisocyanate, cyclohexylene-1,3-diisocyanate, cyclohexylene-1,4-diisocyanate, dicyclohexylmethane-4,4'-diisocyanate, 1,4-bis(isocyanatomethyl)cyclohexane, 1,3-bis(isocyanatomethyl)cyclohexane, isophorone diisocyanate, lysine diisocyanate, hydrogenated tolylene diisocyanate, and hydrogenated xylylene diisocyanate.

Examples of the polyisocyanate also include a tri- or higher functional polyisocyanate (for example, triisocyanate having three functionality and tetraisocyanate having four functionality).

As the tri- or higher functional polyisocyanate, a tri- or higher functional polyisocyanate which is an adduct of an aromatic or alicyclic diisocyanate and a compound having 3 or more active hydrogen groups in one molecule (for example, tri- or higher functional polyol, polyamine, polythiol, or the like) (tri- or higher functional polyisocyanate which is an adduct type), or an aromatic or alicyclic diisocyanate trimer (biuret type or isocyanurate type) is preferable.

Examples of the polyisocyanate also include formalin condensates of benzene isocyanate, polyisocyanates having a polymerizable group, such as methacryloyloxyethyl isocyanate, and lysine triisocyanates.

For the polyisocyanate, the "Polyurethane Resin Handbook" (edited by Keiji Iwata, published by Nikkan Kogyo Shimbun (1987)) can be referred to.

Examples of a commercially available product of the polyisocyanate include TAKENATE (registered trademark) D-102, D-103, D-103H, D-103M2, P49-75S, D-110N, D-120N, D-140N, D-160N, D-127N, D-170N, D-170HN, D-172N, D-177N, D-204, D-165N, and NP1100 (manufactured by Mitsui Chemicals, Inc.); Sumidur N3300, Desmodur (registered trademark) L75, UL57SP, N3200, N3600, N3900, and Z4470BA (manufactured by Sumika Bayer Urethane Co., Ltd.); CORONATE (registered trademark) HL, HX, L, and HK (manufactured by Nippon Polyurethane Industry Co.,Ltd.); P301-75E (manufactured by Asahi Kasei Corporation); Duranate (registered trademark) TPA-100, TKA-100, TSA-100, TSS-100, TLA-100, 24A-100, and TSE-100 (manufactured by Asahi Kasei Corporation); and BURNOCK (registered trademark) D-750 (manufactured by DIC CORPORATION).

Examples of the polyol include aliphatic and aromatic polyhydric alcohols, hydroxypolyester, and hydroxypolyalkylene ether.

Specific examples thereof include polyols described in JP1985-049991A (JP-S60-049991A), ethylene glycol, 1,3-propanediol, 1,4-butanediol, 1,5-pentanediol, 1,6-hexanediol, 1,7-hebutanediol, 1,8-octanediol, propylene glycol, 2,3-dihydroxybutane, 1,2-dihydroxybutane, 1,3-dihydroxybutane, 2,2-dimethyl-1,3-propanediol, 2,4-pentanediol, 2,5-hexanediol, 3-methyl-1,5-pentanediol, 1,4-cyclohexanedimethanol, dihydroxycyclohexane, diethylene glycol, 1,2,6-trihydroxyhexane, 2-phenylpropylene glycol, 1,1,1-trimethylolpropane, hexanetriol, pentaerythritol, pentaerythritol ethylene oxide adduct, glycerin ethylene oxide adduct, glycerin, 1,4-di(2-hydroxyethoxy)benzene, a condensation product of aromatic polyhydric alcohol such as resorcinol dihydroxyethyl ether and alkylene oxide, p-xylylene glycol, m-xylylene glycol, $\alpha,\alpha'$-dihydroxy-p-diisopropylbenzene, 4,4'-dihydroxy-diphenylmethane, 2-(p,p'-dihydroxydiphenylmethyl)benzyl alcohol, ethylene oxide adduct of bisphenol A, and propylene oxide adduct of bisphenol A.

The polyol is preferably used in an amount such that a proportion of a hydroxyl group to 1 mol of an isocyanate group is 0.02 to 2 mol.

Examples of the polyamine include ethylenediamine, trimethylenediamine, tetramethylenediamine, pentamethylenediamine, hexamethylenediamine, p-phenylenediamine, m-phenylenediamine, piperazine, 2-methylpiperazine, 2,5-dimethylpiperazine, 2-hydroxytrimethylenediamine, diethylenetriamine, triethylenetriamine, triethylenetetramine, diethylaminopropylamine, tetraethylenepentamine, and amine adduct of an epoxy compound.

The polyisocyanate can also react with water to form a polymer substance.

Examples of the polyisocyanate, the polyol, and the polyamine include in US3281383A, US3773695A, US3793268A, JP1973-040347B (JP-S48-040347B), JP1974-024159B (JP-S49-024159B), JP1973-080191A (JP-S48-080191A), and JP1973-084086B (JP-S48-084086B), the contents of which are incorporated in the present specification.

An average particle diameter of the microcapsule is preferably 0.1 to 100 $\mu$m in terms of volume average particle diameter. The lower limit value thereof is more preferably 0.3 $\mu$m or more and still more preferably 0.5 $\mu$m or more. The upper limit value thereof is more preferably 10 $\mu$m or less and still more preferably 5 $\mu$m or less. In a case where the average particle diameter (volume average particle diameter) of the microcapsule is 0.1 $\mu$m or more, the core

material in the capsule can be protected more stably. On the other hand, in a case where the average particle diameter (volume average particle diameter) of the microcapsule is 100 μm or less, resolution of the color-formed image is further improved.

The average particle diameter (volume average particle diameter) of the microcapsule can be measured by, for example, a laser analysis and scattering-type particle size distribution analyzer LA950 (manufactured by HORIBA, Ltd.).

In addition, in a case where an average particle diameter of the microcapsule contained in the ultraviolet-sensing member is measured, the average particle diameter (volume average particle diameter) of the microcapsule can be measured with a scanning electron microscope (SEM). Specifically, a surface of the ultraviolet-sensing layer is observed with the SEM at a magnification of 5,000, and the average particle diameter of all microcapsules present in the observed visual field is obtained image analysis. In a case where the microcapsule cannot be observed on the surface, a cross-sectional piece is produced and measured in the same manner as described above.

The above-described microcapsule means a concept including the specific microcapsule A and a microcapsule other than the specific microcapsule A.

(Specific solvent)

The specific microcapsule A contains a specific solvent.

The specific solvent is a solvent having a heteroatom, and examples thereof include an aromatic solvent having a heteroatom and a non-aromatic solvent having a heteroatom.

The "aromatic solvent" means a solvent having an aromatic ring in the molecule. The "non-aromatic solvent" means a solvent having no aromatic ring in the molecule.

· Non-aromatic solvent having heteroatom

The non-aromatic solvent having a heteroatom (also referred to as a non-aromatic solvent X) is not particularly limited as long as it has a heteroatom in the molecule and does not have an aromatic ring.

As the non-aromatic solvent X, a non-aromatic solvent having an aliphatic structure is preferable.

The "having an aliphatic structure" means having a hydrocarbon group having no aromatic ring in the molecule.

The above-described hydrocarbon group having no aromatic ring may be linear, branched, or cyclic. In addition, in the above-described hydrocarbon group having no aromatic ring, a carbon atom in the hydrocarbon group may be substituted with a heteroatom or a carbonyl atom. In addition, the above-described hydrocarbon group may further have a substituent, and the substituent may have a heteroatom.

Examples of the heteroatom include an atom other than a carbon atom and a hydrogen atom, and a nitrogen atom, an oxygen atom, a phosphorus atom, or a sulfur atom is preferable, and an oxygen atom is more preferable.

The number of carbon atoms in the above-described hydrocarbon group is not particularly limited, but is preferably 1 to 50, more preferably 6 to 50, and still more preferably 8 to 30.

As the non-aromatic solvent X, one or more solvents selected from the group consisting of an aliphatic carboxylic acid, a fatty acid ester, an ether-based solvent, an alcohol-based solvent, an amide-based solvent, and a ketone-based solvent are preferable. From the viewpoint of promoting the coloring reaction, an alcohol-based solvent is preferable, and from the viewpoint of encapsulation reaction suitability and safety, an aliphatic carboxylic acid, a fatty acid ester, an ether-based solvent, an amide-based solvent, or a ketone-based solvent is preferable.

Examples of the aliphatic carboxylic acid include oleic acid, dimethyl succinate, diethyl succinate, and methyl laurate.

Examples of the fatty acid ester include an unsaturated fatty acid ester and a saturated fatty acid ester. Specific examples thereof include natural animal and vegetable oils, such as soybean oil, corn oil, cotton seed oil, rapeseed oil, olive oil, coconut oil, castor oil, and fish oil.

In addition, examples of the fatty acid ester include an aliphatic carboxylic acid ester, an aliphatic sulfonic acid ester, and an aliphatic phosphoric acid ester, and an aliphatic phosphoric acid ester is preferable. Specific examples thereof include tri(2-ethylhexiel)phosphate.

Examples of the ether-based solvent include propylene glycol monobutyl ether.

Examples of the ketone-based solvent include cyclohexanone.

As the alcohol-based solvent, from the viewpoint that it facilitates the encapsulation reaction, a long-chain alkyl alcohol is preferable, and a long-chain alkyl monoalcohol is more preferable, and a long-chain alkyl monoalcohol having 6 to 20 carbon atoms is particularly preferable. Examples thereof include octanol.

Examples of the amide-based solvent include N,N-diethyldodecaneamide.

A boiling point of the non-aromatic solvent X is preferably 100°C or higher, more preferably 120°C or higher, and still more preferably 140°C or higher. The upper limit thereof is not particularly limited, but is preferably 500°C or

lower. In a case where the boiling point of the non-aromatic solvent X is 100°C or higher, the specific solvent is likely to remain without being excluded from the specific microcapsule A in a heating step such as a microencapsulation reaction.

A molecular weight of the non-aromatic solvent X is not particularly limited, and is often 100 or more, preferably 150 or more. The upper limit thereof is not particularly limited, but is preferably 1,000 or less, more preferably 600 or less, and still more preferably 500 or less.

The non-aromatic solvent X may be used alone or in combination of two or more kinds thereof.

A content of the non-aromatic solvent X in the specific microcapsule A is preferably 1% to 100% by mass, more preferably 10% to 100% by mass, and still more preferably 25% to 100% by mass with respect to the total mass of the solvent.

The type, content, and compositional ratio of the non-aromatic solvent X can be analyzed by extracting the ultraviolet-sensing layer with acetone, concentrating the obtained filtrate, and performing Gas Chromatography Mass Spectrometry (GC-MS) analysis.

· Aromatic solvent having heteroatom

The aromatic solvent having a heteroatom (also referred to as an aromatic solvent Y) is not particularly limited as long as it has a heteroatom in the molecule and has an aromatic ring.

Examples of the aromatic ring included in the aromatic solvent Y include an aromatic hydrocarbon ring and an aromatic heterocyclic ring, and an aromatic hydrocarbon ring is preferably used.

The above-described aromatic hydrocarbon ring may be a monocyclic ring or a fused polycyclic ring, but a monocyclic ring is preferable.

In addition, the above-described aromatic hydrocarbon ring may have a substituent. In a case where the above-described aromatic hydrocarbon ring has a plurality of substituents, the substituents may be bonded to each other to form an alicyclic ring. In addition, the above-described aromatic hydrocarbon ring may have an aliphatic structure.

The number of carbon atoms in the above-described aromatic hydrocarbon ring is not particularly limited, but is preferably 6 to 30, more preferably 6 to 18, and still more preferably 6 to 10.

Examples of the monocyclic aromatic hydrocarbon ring include a benzene ring.

Examples of the polycyclic aromatic hydrocarbon ring include a naphthalene ring.

The above-described aromatic heterocyclic ring may be a monocyclic ring or a polycyclic ring.

In addition, the above-described aromatic heterocyclic ring may have a substituent.

The number of aromatic rings in the aromatic solvent is not particularly limited, and may be one or two or more. In a case of including two or more aromatic rings, the two aromatic rings may form a polycyclic structure formed by bonding substituents which may exist on each aromatic ring to each other (however, excluding a fused polycyclic structure).

Examples of the aromatic solvent Y include an aromatic solvent having an aromatic heterocyclic ring in the molecule, and an aromatic solvent having a heteroatom and an aromatic hydrocarbon ring in the molecule.

Examples of the heteroatom in the aromatic solvent Y include an atom other than a carbon atom and a hydrogen atom, and a nitrogen atom, an oxygen atom, a sulfur atom, or a phosphorus atom is preferable, and an oxygen atom or a phosphorus atom is more preferable. From the viewpoint that, while ensuring the transmittance at the wavelength of 222 nm, it promotes the coloring reaction and has excellent sensitivity at the wavelength of 222 nm, the aromatic solvent Y preferably includes at least one selected from the group consisting of a carboxylate linking group, a sulfonate linking group, a phosphate linking group, a carbonyl linking group, and a sulfone linking group.

Specific examples of the aromatic solvent Y include substituted or unsubstituted benzenesulfonate esters such as methyl benzenesulfonate, ethyl benzenesulfonate, methyl toluenesulfonate, and ethyl toluenesulfonate; substituted or unsubstituted phthalate diesters such as dimethyl phthalate, diethyl phthalate, dibutyl phthalate, dipentyl phthalate, dihexyl phthalate, and dicyclohexyl phthalate; and aromatic phosphates such as triphenyl phosphate (TPP), tricresyl phosphate (TCP), trixylenyl phosphate (TXP), cresyl diphenyl phosphate (CDP), 2-ethylhexyldiphenyl phosphate (EHDP), t-butylphenyl diphenyl phosphate (t-BDP), bis-(t-butylphenyl)phenyl phosphate (BBDP), tris-(t-butylphenyl) phosphate (TBDP), isopropylphenyl diphenyl phosphate (IPP), bis-(isopropylphenyl)diphenyl phosphate (BIPP), and tris-(isopropylphenyl) phosphate (TIPP).

The aromatic solvent Y may be used alone or in combination of two or more kinds thereof.

The boiling point, molecular weight, and content of the aromatic solvent Y are the same as those of the non-aromatic solvent X described above, and suitable aspects thereof are also the same.

The type, content, and compositional ratio of the solvent can be analyzed by the same method as the analysis method for the non-aromatic solvent X described above.

The specific solvent preferably includes one or more solvents having a boiling point of 100°C or higher, and it is more preferable that all specific solvents contained in the specific microcapsule A have a boiling point of 100°C or higher. The upper limit of the boiling point of the specific solvent is preferably 500°C or lower.

It is preferable that all solvents contained in the specific microcapsule A have a boiling point of 100°C or higher. The upper limit of the boiling point of all solvents is preferably 500°C or lower.

(Color-forming agent)

The specific microcapsule A contains a color-forming agent. Examples of the color-forming agent contained in the specific microcapsule A include the same type as the color-forming agent contained in the ultraviolet-sensing layer described above, and a suitable aspect thereof is also the same.

(Photoactivator)

The specific microcapsule A contains a photoactivator. Examples of the photoactivator contained in the specific microcapsule A include the same type as the photoactivator contained in the ultraviolet-sensing layer described above, and a suitable aspect thereof is also the same.

(Light stabilizer)

The specific microcapsule A preferably contains a light stabilizer.
The light stabilizer is not particularly limited as long as it is a material which stabilizes with light, but it is preferably a light stabilizer which acts as a so-called free-radical scavenger, trapping free radicals of the activated photoactivator.
The light stabilizer may be used alone or in combination of two or more kinds thereof.
Examples of the light stabilizer include polyhydric phenols such as 2,5-bis(1,1,3,3-tetramethylbutyl)hydroquinone, hydroquinone, catechol, resorcinol, and hydroxyhydroquinone; aminophenols such as o-aminophenol and p-aminephenol.
A content ratio of the light stabilizer to the photoactivator (light stabilizer/photoactivator (molar ratio)) is preferably 0.0001 to 10 and more preferably 0.0002 to 5.

(Reducing agent)

The specific microcapsule A may contain a reducing agent.
The reducing agent has a function of inactivating the photooxidant.
In a case where the specific microcapsule A contains a reducing agent, a rapid change in color optical density of the ultraviolet-sensing layer due to ultraviolet irradiation can be suppressed, and the color optical density can be easily changed according to the amount of ultraviolet irradiation. The reducing agent may also function as an antioxidant.
The reducing agent may be used alone or in combination of two or more kinds thereof.
Examples of the reducing agent include a cyclic phenylhydrazide compound. Specific examples thereof include 1-phenylpyrazolidin-3-one, 1-phenyl-4-methylbyrazolidin-3-one, 1-phenyl-4,4-dimethylpyrazolidin-3-one, 3-methyl-1-p-sulfophenyl-2-pyrazolin-5-one, 3-methyl-1-phenyl-2-byrazolin-5-one, and 4-hydroxymethyl-4-methyl-1-phenyl-3-pyrazolidinone (Dimezone S, manufactured by Daito Chemical Co., Ltd.).
As the reducing agent, reducing agents described in paragraphs 0072 to 0075 of WO2016/017701A can be referred to, the contents of which are incorporated in the present specification.

(Ultraviolet absorber)

The specific microcapsule A may contain an ultraviolet absorber.
Examples of the ultraviolet absorber include a benzotriazole compound (an ultraviolet absorber having a benzotriazole structure), a benzophenone compound, a triazine compound, and a benzodithiol compound.
Among these, from the viewpoint that the sensitivity at the wavelength of 222 nm is more excellent, the ultraviolet absorber preferably has a small absorption at the wavelength of 222 nm, and specifically, a triazine compound, a benzophenone compound, or a benzodithiol compound is preferably used.
In addition, it is preferable that the specific microcapsule A does not contain a benzotriazole compound having a large absorption at the wavelength of 222 nm. In a case where the specific microcapsule A contains a benzotriazole compound, a content of the benzotriazole compound is preferably 1% by mass or less, and more preferably 0.5% by mass or less with respect to the total mass of the photoactivator. The lower limit thereof is not limited, but is, for example, 0.0001% by mass or more.
In addition, the content of the benzotriazole compound is preferably 1% by mass or less, and more preferably 0.5% by mass or less, with respect to the total mass of the color-forming agent. The lower limit thereof is not limited, but is, for example, 0.0001% by mass or more.

The ultraviolet absorber may be used alone or in combination of two or more kinds thereof.
Examples of a commercially available triazine compound include ADK STAB LA-F70 (manufactured by ADEKA Corporation); Tinuvin 1577 ED and Tinuvin 1600 (manufactured by BASF); 2,4-Bis(2,4-dimethylphenyl)-6-(2-hydroxy-4-n-octyloxyphenyl) -1,3,5-triazine, 2-(2,4-Dihydroxyphenyl)-4,6-diphenyl-1,3,5-triazine, and Ethylhexyl Triazone (manufactured by TOKYO CHEMICAL INDUSTRY CO., LTD.).
Examples of a commercially available benzophenone compound include Chimassorb 81 and Chimassorb 81 FL (manufactured by BASF).
Examples of the benzodithiol compound include compounds described in WO2019/159570A.

(Other components)

The specific microcapsule A may contain, as necessary, one or more additives such as a wax, a solvent other than the specific solvent, and an odor suppressant, in addition to the above-described components.

<Production method of specific microcapsule A>

A production method of the specific microcapsule A is not particularly limited, and examples thereof include known methods such as an interfacial polymerization method, an internal polymerization method, a phase separation method, an external polymerization method, and a coacervation method.
As an example of the production method of the specific microcapsule A, a method including the following emulsification step and encapsulation step can be mentioned. In the encapsulation step, it is preferable to form a resin wall (capsule wall) by an interfacial polymerization method.

Emulsification step: a step of mixing the color-forming agent, the photoactivator, the specific solvent, and an emulsifier in water to prepare an emulsified liquid
Encapsulation step: a step of forming a resin wall (capsule wall) around an oil droplet including the color-forming agent, the photoactivator, and the specific solvent in the emulsified liquid obtained in the emulsification step to encapsulate the oil droplet

Hereinafter, the interfacial polymerization method as the example of the production method of the specific microcapsule A, in which the capsule wall is polyurea or polyurethane urea, will be described.
The interfacial polymerization method is preferably an interfacial polymerization method including a step (emulsification step) of dispersing an oil phase, which contains a photoactivator, a specific solvent, a solvent including an aliphatic structure with a boiling point of lower than 100°C (hereinafter, also referred to as "solvent for producing a capsule"), a color-forming agent, and a capsule wall material (for example, polyisocyanate), in a water phase which contains an emulsifier to prepare an emulsified liquid; and a step (encapsulation step) of forming a capsule wall by polymerizing the capsule wall material at an interface between the oil phase and the water phase, and forming a microcapsule containing the photoactivator selected from the oxidant and the photoacid generator, the specific solvent, and the color-forming agent.
In the above-described emulsification step, the solvent for producing a capsule is usually a component which can be added for the purpose of improving solubility of the core material in the solvent. The solvent for producing a capsule does not include an aromatic ring in the molecule. In addition, the solvent for producing a capsule is removed by a drying treatment in a method for forming an ultraviolet-sensing layer, which will be described later. Therefore, it is preferable that the microcapsule in the ultraviolet-sensing member does not contain the solvent for producing a capsule.
The solvent for producing a capsule is not particularly limited, and examples thereof include ethyl acetate (boiling point: 77°C), isopropyl acetate (boiling point: 89°C), methyl ethyl ketone (boiling point: 80°C), and methylene chloride (boiling point: 40°C).
The solvent for producing a capsule may be used alone or in combination of two or more kinds thereof.
In addition, the type of the emulsifier used in the above-described emulsification step is not particularly limited, and examples thereof include a dispersant and a surfactant.
Examples of the dispersant include water-soluble polymers selected from known anionic polymers, non-ionic polymers, and amphoteric polymer, and specific examples thereof include polyvinyl alcohol, gelatin, and cellulose derivative. Among these, polyvinyl alcohol is preferably used.
The dispersant may be used alone or in combination of two or more kinds thereof.
The surfactant is preferably an anionic or non-ionic surfactant, and examples thereof include alkylbenzenesulfonates (such as sodium dodecylbenzenesulfonate and ammonium dodecylbenzenesulfonate), alkylsulfonates (such as sodium lauryl sulfate and dioctyl sodium sulfosuccinate), polyalkylene glycols (such as polyoxyethylene nonylphenyl ether).

The surfactant may be used alone or in combination of two or more kinds thereof.

In addition, as another production method of the specific microcapsule A, methods described in US3726804A and US3796696A can also be referred to. The contents thereof are incorporated in the present specification.

A content of the specific microcapsule A in the ultraviolet-sensing layer is not particularly limited, but is preferably 50% to 99% by mass and more preferably 60% to 90% by mass with respect to the total mass of the ultraviolet-sensing layer.

A content (coating amount of solid content) of the specific microcapsule A in the ultraviolet-sensing layer is also preferably 0.1 to 30 $g/m^2$. The lower limit value thereof is preferably 0.5 $g/m^2$ or more, and more preferably 1 $g/m^2$ or more. The upper limit value thereof is preferably 25 $g/m^2$ or less, and more preferably 20 $g/m^2$ or less.

The ultraviolet-sensing layer may contain a component other than the above-described specific microcapsule A.

Examples of other components include a polymer binder, a reducing agent, a light stabilizer, a crosslinking agent, a sensitizer, an ultraviolet absorber, a surfactant, and a colorant.

The compound used as another component may be used alone or in combination of two or more kinds thereof.

Examples of the polymer binder include various emulsions such as polyvinyl alcohol, methylcellulose, ethylcellulose, carboxymethylcellulose, hydroxypropylcellulose, gum arabic, gelatin, polyvinylpyrrolidone, casein, styrene-butadiene latex, acrylonitrile-butadiene latex, polyacrylic acid ester, polyacrylate, and ethicine-vinyl acetate copolymer.

In addition, as the polymer binder, polymer binders described in paragraph 0078 of JP2017-167155A can be referred to, the contents of which are incorporated in the present specification.

The polymer binder may be crosslinked. In other words, the polymer binder may be a crosslinked binder.

The crosslinking agent is not particularly limited, and for example, glyoxazole can be used. In addition, a crosslinking agent described in paragraph 0079 of JP2017-167155A can also be referred to. The contents thereof are incorporated in the present specification.

As the reducing agent, the sensitizer, and the surfactant, description in lower left column of page 9 to upper left column of page 10 in JP1989-207741A (JP-H1-207741A) and in paragraphs 0038, 0039, and 0048 to 0059 of JP2004-233614A can be referred to, the contents of which are incorporated in the present specification.

In addition, as the reducing agent, the light stabilizer, the ultraviolet absorber, and the surfactant, the reducing agent, the light stabilizer, the ultraviolet absorber, and the surfactant, which can be contained in the specific microcapsule A, can also be used.

By using the colorant in combination with the above-described color-forming agent, it is easy to control the tint.

Examples of the colorant include dyes and pigments. The pigment is not particularly limited, and examples thereof include yellow pigments described in paragraphs 0018 to 0022 of WO2016/017701A and inorganic particles as a white pigment.

The colorant may be contained in the above-described specific microcapsule A, or may be contained in the ultraviolet-sensing layer outside the specific microcapsule A. In a case where the specific microcapsule A contains a colorant, the colorant is preferably a colorant which is soluble in the solvent.

A mass (coating amount of solid content) per unit area of the ultraviolet-sensing layer is not particularly limited, but for example, is preferably 0.1 to 30 $g/m^2$, more preferably 0.5 to 25 $g/m^2$, and still more preferably 1 to 10 $g/m^2$.

A thickness of the ultraviolet-sensing layer is preferably 0.1 to 30 $\mu$m, more preferably 0.5 to 25 $\mu$m, and still more preferably 1 to 10 $\mu$m.

<Method for forming ultraviolet-sensing layer>

A method for forming the above-described ultraviolet-sensing layer is not particularly limited, and examples thereof include known methods.

Examples thereof include a method of applying a dispersion liquid for forming an ultraviolet-sensing layer, which contains the specific microcapsule A, onto a support, and as necessary, drying the coating film.

The dispersion liquid for forming an ultraviolet-sensing layer contains the specific microcapsule A. The microcapsule dispersion liquid obtained by the above-described interfacial polymerization method may be used as the dispersion liquid for forming an ultraviolet-sensing layer.

The dispersion liquid for forming an ultraviolet-sensing layer may contain other components which may be contained in the above-described ultraviolet-sensing layer.

The method of applying the dispersion liquid for forming an ultraviolet-sensing layer is not particularly limited, examples of a coating machine used for the applying include an air knife coater, a rod coater, a bar coater, a curtain coater, a gravure coater, an extrusion coater, a die coater, a slide bead coater, and a blade coater.

After the dispersion liquid for forming an ultraviolet-sensing layer is applied onto the support, the coating film may be subjected to a drying treatment, as necessary. Examples of the drying treatment include a heating treatment.

Although the method for forming the ultraviolet-sensing layer on the support has been described, the present invention is not limited to the above-described aspect. For example, after forming the ultraviolet-sensing layer on a

temporary support, the temporary support may be peeled off to form the ultraviolet-sensing member including the ultraviolet-sensing layer.

The temporary support is not particularly limited as long as it is a peelable support.

<<Other layers>>

The ultraviolet-sensing member may include a layer other than the support and the ultraviolet-sensing layer described above.

Examples of other layers include a reflective layer, a glossy layer, a filter layer, and a sensitivity-adjusting layer.

<Reflective layer>

The ultraviolet-sensing member may further include a reflective layer.

In a case where the ultraviolet-sensing layer includes a reflective layer, since ultraviolet rays irradiated to the ultraviolet-sensing member can be reflected by the layer having ultraviolet reflectivity, scattering of the ultraviolet rays inside the ultraviolet-sensing member can be suppressed, and detection accuracy of the amount of ultraviolet irradiation can be further improved.

A reflectivity of the reflective layer with respect to light having a wavelength of 200 to 380 nm is preferably 10% or more, and more preferably 50% or more. The reflectivity can be measured, for example, by diffusion reflection measurement using an ultraviolet-visible spectrophotometer (UV-2700, Shimadzu Corporation).

In a case where the support is disposed adjacent to the reflective layer, an adhesive layer may be provided between the support and the reflective layer.

As the reflective layer, the adhesive layer, and manufacturing methods thereof, the reflective layer, the adhesive layer, and manufacturing methods thereof, which are described in paragraphs 0082 to 0091 of WO2016/017701A, can be referred to. The contents thereof are incorporated in the present specification.

<Glossy layer>

The ultraviolet-sensing member may further include a glossy layer.

In a case where the ultraviolet-sensing layer includes a glossy layer, visibility of front and back surfaces can be improved.

As the glossy layer and a method for producing the glossy layer, glossy layers and method for producing the glossy layer, described in paragraphs 0092 to 0094 of WO2016/017701A, can be referred to, the contents of which are incorporated in the present specification.

<Filter layer>

It is preferable that the ultraviolet-sensing member further includes a filter layer.

The filter layer is a layer which selectively transmits light having a specific wavelength. Here, the "selectively transmits light having a specific wavelength" means transmitting the light having a specific wavelength and shielding other lights. For example, a transmittance of light having a wavelength to be transmitted is preferably 70% or more, more preferably 80% or more, and still more preferably 90% or more. A transmittance of light having a wavelength to be shielded is preferably 30% or less, more preferably 20% or less, and still more preferably 10% or less.

The filter layer is preferably a filter layer which shields light having a wavelength of 300 nm or more, and more preferably a filter layer which shields light having a wavelength of more than 230 nm. An ultraviolet band pass filter, a filter containing a dielectric, or the like is preferably used.

Spectral characteristics of the filter layer and the sensitivity-adjusting layer described later can be measured using, for example, an ultraviolet-visible spectrophotometer (UV-2700, Shimadzu Corporation).

The filter layer preferably has an ultraviolet absorber from the viewpoint of shielding light having a wavelength other than the specific wavelength. As the ultraviolet absorber, a known ultraviolet absorber can be used. In addition, from the viewpoint of shielding light having a wavelength of more than 230 nm, it is preferable to contain an ultraviolet absorber which can be contained in the specific microcapsule A.

As the filter layer and a method for producing the filter layer, filter layers and method for producing the filter layer, described in paragraphs 0016 to 0026 of WO2016/017701A, can be referred to, the contents of which are incorporated in the present specification.

<Sensitivity-adjusting layer>

In a case where the ultraviolet-sensing member includes the filter layer, a sensitivity-adjusting layer may be further provided on a surface of the filter layer.
As the sensitivity-adjusting layer and a method for producing the glossy layer, sensitivity-adjusting layers and method for producing the sensitivity-adjusting layer, described in paragraphs 0095 to 0109 of WO2016/017701A, can be referred to, the contents of which are incorporated in the present specification.

[Second embodiment]

A second embodiment of the ultraviolet ray-sensitive member according to the present invention is an ultraviolet-sensing member including an ultraviolet-sensing layer which contains a microcapsule (hereinafter, also referred to as "specific microcapsule B") containing a photoactivator, a color-forming agent, and a solvent, in which a capsule wall of the microcapsule contains one or more resins selected from the group consisting of a polyurea having an aliphatic ring, a polyurethane urea having an aliphatic ring, and a polyurethane having an aliphatic ring, and a peak surface area proportion X obtained by a peak surface area proportion calculation method X is 30% or less.
Peak surface area proportion calculation method X: a liquid chromatography measurement is performed for each of a first solution which is obtained by cutting out two test pieces of the same size from the ultraviolet-sensing member and immersing one of the test pieces in n-propanol for 7 days and a second solution obtained by immersing the other test piece in n-propanol for 1 hour, and a proportion of a peak area of the color-forming agent in the second solution to a peak area of the color-forming agent in the first solution is calculated as the peak surface area proportion X
In the second embodiment, any one of the difference between C1 and C2, the difference between Y1 and Y2, or the difference between M1 and M2 described above can be easily controlled to 0.20 or more.
The second embodiment has the same configuration as that of the first embodiment, except that the specific microcapsule B is used and the peak surface area proportion X shows a predetermined value. Therefore, descriptions other than the specific microcapsule B and the peak surface area proportion X are omitted.
The present inventor has found that the reason why the desired effect cannot be obtained in the ultraviolet-sensing member in the related art is that, first, the material of the capsule wall of the microcapsule usually has an aromatic group, and in such cases, light having a wavelength of 222 nm is absorbed by the capsule wall, and less light reaches the photoactivator, resulting in inferior sensitivity. Therefore, in the present invention, the sensitivity is further improved by using a predetermined resin containing an aliphatic ring, which has excellent transmittance at the wavelength of 222 nm.
In addition, the present inventor has found that, in the ultraviolet-sensing member in the related art, the encompassed substances of the microcapsule are likely to leak, and as a result, the sensitivity at the wavelength of 222 nm is inferior. In the microcapsule, the color-forming agent is present in a liquid phase called a solvent, so that the action of the photoactivator on the color-forming agent is good. However, it is considered that, in a case where the encompassed substances leak, the color-forming agent may be precipitated from the solvent, and the action of the photoactivator on the color-forming agent is deteriorated, resulting in deterioration of the color formability. It is presumed that the reason why the encompassed substances are likely to leak in the prior art is that a polymerization of the resin constituting the capsule wall of the microcapsule does not sufficiently proceed. Therefore, it has been found that, in a case where the peak surface area proportion X described below is within a predetermined range, the leakage of the encompassed substances from the microcapsule can be suppressed, and as a result, a more excellent effect can be obtained.

<<Peak surface area proportion X>>

The peak surface area proportion X is a value calculated by the following peak surface area proportion calculation method X. The peak surface area proportion X is an index of easiness of leakage of the color-forming agent from the microcapsule, and in a case where this value is small, it means that the color-forming agent is unlikely to leak from the microcapsule. More specifically, in the following method X, first, the ultraviolet-sensing member is immersed in n-propanol for 7 days to elute the color-forming agent contained in the microcapsule contained in the ultraviolet-sensing member, thereby obtaining a first solution containing the color-forming agent. This first solution is used as a reference for a second solution described below. Next, the ultraviolet-sensing member is immersed in n-propanol for 1 hour to elute the color-forming agent contained in the microcapsule contained in the ultraviolet-sensing member, thereby obtaining a second solution containing the color-forming agent. In a case where the amount of the color-forming agent eluted in the second solution is large with respect to the amount of the color-forming agent eluted in the first solution (in other words, the peak surface area proportion X is large), it means that the color-forming agent contained in the microcapsule is eluted in a short time, and that the color-forming agent easily leaked from the microcapsule. On the other hand, in a case where the amount of the color-forming agent eluted in the second solution is small with respect to the amount of the color-forming agent eluted in the first solution (in other words, the peak surface area proportion X is small), it means that the

color-forming agent contained in the microcapsule is less likely to elute and the color-forming agent is less likely to leak from the microcapsule.

Peak surface area proportion calculation method X: a liquid chromatography measurement is performed for each of a first solution which is obtained by cutting out two test pieces of the same size from the ultraviolet-sensing member and immersing one of the test pieces in n-propanol for 7 days and a second solution obtained by immersing the other test piece in n-propanol for 1 hour, and a proportion of a peak area of the color-forming agent in the second solution to a peak area of the color-forming agent in the first solution is calculated as the peak surface area proportion X

Hereinafter, the procedure of the calculation method of the peak surface area proportion X will be described in detail.

First, two test pieces of the same size (circular shape having a diameter of 2 cm) are cut out from the ultraviolet-sensing member to be measured.

Next, one of the cut-out test pieces is immersed in n-propanol (20 ml) for 7 days at room temperature (20°C to 25°C), and the obtained solution is used as the first solution. During the immersion, a stirring treatment is not performed and the mixture is allowed to stand. Usually, after 7 days, the test piece is taken out from the first solution. The n-propanol is prevented from volatilizing during the immersion.

In addition, one of the cut-out test pieces is immersed in n-propanol (20 ml) for 1 hour at room temperature (20°C to 25°C), and the obtained solution is used as the second solution. During the immersion, a stirring treatment is not performed and the mixture is allowed to stand. Usually, after 1 hour, the test piece is taken out from the second solution.

Next, a liquid chromatography measurement of the obtained first solution and second solution is performed. In a case of performing the liquid chromatography measurement, injection amounts of the first solution and the second solution are the same.

Measurement conditions of the liquid chromatography measurement are as follows.

Equipment: Nexera manufactured by Shimadzu Corporation
Column: Capcell pak C18 UG-120
Eluent: water/methanol
Oven: 40°C
Injection: 5 μL
Detection: maximal absorption wavelength of color-forming agent to be detected
Flow Rate: 0.2 mL/min

Next, a peak surface area of the color-forming agent in the first solution (hereinafter, also referred to as "peak surface area 1") is obtained from the liquid chromatography measurement result of the first solution, and further, a peak surface area of the color-forming agent in the second solution (hereinafter, also referred to as "peak surface area 2") is obtained from the liquid chromatography measurement result of the second solution, a peak surface area proportion X, which is a proportion of the peak surface area 2 to the peak surface area 1 {(peak surface area 2/peak surface area 1) × 100}, is calculated.

The peak surface area proportion X is 30% or less, and from the viewpoint that the effect of the present invention is more excellent, it is preferably 20% or less and more preferably 10% or less. The lower limit thereof is not particularly limited, but is preferably 0% and is often 1% or more.

The specific microcapsule B usually includes a core portion and a capsule wall for encompassing a core material (encompassed substance (hereinafter, also referred to as an encompassed component)) forming the core portion.

The specific microcapsule contains, as the core material (encompassed component), the photoactivator and the color-forming agent.

Examples of one suitable aspect of the specific microcapsule B include an aspect in which the photoactivator is the photooxidant and the color-forming agent is the color-forming agent which forms color by being oxidized.

In addition, examples of another suitable aspect of the specific microcapsule B include an aspect in which the photoactivator is the photoacid generator and the color-forming agent is the color-forming agent which forms color by action of acid.

(Capsule wall)

The capsule wall of the specific microcapsule B contains one or more resins selected from the group consisting of a polyurea having an aliphatic ring, a polyurethane urea having an aliphatic ring, and a polyurethane having an aliphatic ring (hereinafter, these are collectively referred to as "specific resin").

It is preferable that the capsule wall of the specific microcapsule is substantially composed of the specific resin. The term "substantially composed of the specific resin" means that a content of the specific resin with respect to the total mass of the capsule wall is 90% by mass or more, preferably 100% by mass. That is, it is preferable that the capsule

wall of the specific microcapsule is composed of the specific resin.

The aliphatic ring included in each specific resin may have a monocyclic structure or a polycyclic structure. The number of rings included in the polycyclic structure is not particularly limited, and examples thereof include 2 and 3.

The number of carbon atoms included in the aliphatic ring is not particularly limited, and is preferably 6 to 20 and more preferably 6 to 12.

The aliphatic ring may be a saturated aliphatic ring or an unsaturated aliphatic ring.

Examples of the aliphatic ring include a cycloalkane ring (for example, a cyclohexane ring), an adamantane ring, and a norbornene ring.

From the viewpoint that the effect of the present invention is more excellent, the capsule wall of the specific microcapsule B preferably has a structure derived from polyisocyanate having an aliphatic ring. Among these, it is preferable that the capsule wall of the specific microcapsule B contains one or more resins selected from the group consisting of a polyurea having a structure derived from a polyisocyanate having an aliphatic ring, a polyurethane urea having a structure derived from a polyisocyanate having an aliphatic ring, and a polyurethane having a structure derived from a polyisocyanate having an aliphatic ring.

The description of the aliphatic ring included in the polyisocyanate having an aliphatic ring is as described above.

The number of alicyclic rings in the polyisocyanate having an aliphatic ring is not particularly limited, may be 1 or 2 or more, and is preferably 1 to 3.

The number of isocyanate groups included in the polyisocyanate having an aliphatic ring is not particularly limited, and is preferably 2 to 10, more preferably 2 to 5, and still more preferably 2 to 3.

The capsule wall of the specific microcapsule B may further have an aromatic ring. That is, the capsule wall of the specific microcapsule B may contain one or more resins selected from the group consisting of a polyurea having an aliphatic ring and an aromatic ring, a polyurethane urea having an aliphatic ring and an aromatic ring, and a polyurethane having an aliphatic ring and an aromatic ring.

Examples of the aromatic ring include an aromatic hydrocarbon ring and an aromatic heterocyclic ring, and an aromatic hydrocarbon is preferably used.

The above-described aromatic hydrocarbon ring may be a monocyclic ring or a fused polycyclic ring.

The number of carbon atoms in the above-described aromatic hydrocarbon ring is not particularly limited, but is preferably 6 to 30, more preferably 6 to 18, and still more preferably 6 to 10.

Examples of the monocyclic aromatic hydrocarbon ring include a benzene ring.

Examples of the fused polycyclic aromatic hydrocarbon ring include a naphthalene ring.

Examples of the polyisocyanate and the polyol used include the polyisocyanate and the polyol described in the first embodiment above.

Examples of the color-forming agent contained in the specific microcapsule B include the same type as the color-forming agent contained in the ultraviolet-sensing layer described above, and a suitable aspect thereof is also the same.

Examples of the photoactivator contained in the specific microcapsule B include the same type as the photoactivator contained in the ultraviolet-sensing layer described above, and a suitable aspect thereof is also the same.

The specific microcapsule B contains a solvent.

The type of the solvent is not particularly limited, and examples thereof include an aromatic solvent and a non-aromatic solvent.

Examples of the aromatic solvent include the aromatic solvent Y described in the first embodiment. Examples of the non-aromatic solvent include the non-aromatic solvent X described in the first embodiment.

The solvent contained in the specific microcapsule is preferably a solvent compatible with n-propanol. The above-described compatible solvent means a solvent in which phase separation does not occur in a case where the same amount of the solvent is mixed with n-propanol.

The specific microcapsule B may contain, as necessary, one or more additives such as a reducing agent, a light stabilizer, a wax, an ultraviolet absorber, and an odor suppressant, in addition to the above-described components. Among these, it is preferable that the light stabilizer is contained.

Details of the various components (for example, a reducing agent and a light stabilizer) are as described in the first embodiment.

[Other embodiments]

In the first embodiment and the second embodiment described above, the aspect in which the microcapsule is used has been described, but an aspect in which the microcapsule is not used may be used. For example, the ultraviolet-sensing member may include an ultraviolet-sensing layer containing a predetermined binder resin and a color-forming agent dispersed and/or melted in the binder resin. The above-described ultraviolet-sensing layer may further contain a photoactivator. In addition, the above-described ultraviolet-sensing layer may contain the above-described

other components (reducing agent, light stabilizer, crosslinking agent, sensitizer, ultraviolet absorber, surfactant, colorant, and the like).

In addition, a molded body in which the specific microcapsules are kneaded into the binder resin may be used.

It is preferable that the binder resin includes either a water-soluble binder resin or a water-insoluble binder resin.

Examples of the binder resin include cellulose resins such as methylcellulose, ethylcellulose, carboxymethylcellulose, and hydroxypropylcellulose, gum arabic, gelatin, polyvinylpyrrolidone, casein, styrene-butadiene copolymer, acrylonitrile-butadiene copolymer, polyvinyl acetate, acrylic resin, polyvinyl chloride, and ethylene-vinyl acetate copolymer. From the viewpoint of excellent sensitivity at a wavelength of 222 nm, as the binder resin, a binder resin having substantially no aromatic group is preferable, and a cellulose resin or an acrylic resin is preferable.

From the viewpoint that the effect of the present invention is more excellent, the binder is preferably a resin including a hydroxyl group. Examples of the resin including a hydroxyl group include a cellulose resin, polyvinyl alcohol, and polyvinyl butyral.

From the viewpoint that it does not form color without irradiation and has excellent storage stability, it is preferable that the binder has a low acid value. It is more preferably 0 to 50 mgKOH/g, and still more preferably 0 to 20 mgKOH/g.

It is preferable that the binder has substantially no aromatic ring group because excessive absorption of light having a wavelength of 222 nm is suppressed. The "substantially no aromatic ring group" means that a content of the aromatic ring group is preferably 0% to 1% by mass and more preferably 0% to 0.1% by mass with respect to the total mass of the binder.

In addition, as the binder resin, polymer binders described in paragraph 0078 of JP2017-167155A can be referred to, the contents of which are incorporated in the present specification.

The binder resin may be used alone or in combination of two or more kinds thereof.

The binder resin may be crosslinked. In other words, the binder resin may be a crosslinked binder resin.

The crosslinking agent is not particularly limited, and for example, glyoxazole can be used. In addition, a crosslinking agent described in paragraph 0079 of JP2017-167155A can also be referred to. The contents thereof are incorporated in the present specification.

[Ultraviolet-sensing kit]

In addition, the present invention also relates to an ultraviolet-sensing kit including the above-described ultraviolet-sensing member.

The ultraviolet-sensing kit includes at least the above-described ultraviolet-sensing member.

A specific configuration of the ultraviolet-sensing kit is not particularly limited, and examples thereof include an aspect of including the ultraviolet-sensing member and other elements selected from the group consisting of a member having a filter layer which selectively transmits light having a specific wavelength (preferably a filter sheet which shields light having a wavelength of 300 nm or more, and more preferably a filter sheet which shields light having a wavelength of more than 230 nm), a light shielding bag (ultraviolet cut bag), a sample judgment, a limit sample (calibration sheet), a condensing jig such as a lens and a concave mirror, and a holding member which holds the ultraviolet-sensing member.

The above-described holding member may have an opening portion for irradiating the held ultraviolet-sensing member with ultraviolet rays, or the holding member and a determination sample may be integrated.

## Examples

Hereinafter, the features of the present invention will be more specifically described using Examples and Comparative Examples. The materials, the amounts of materials used, the proportions, the treatment details, the treatment procedure, and the like shown in Examples below may be modified as appropriate as long as the modifications do not depart from the spirit of the present invention. Accordingly, the scope of the present invention should not be construed as being limited by the specific examples given below.

[Production and evaluation of ultraviolet-sensing member]

[Example 1]

A mixed solution 1 having the following composition was added to a 5% by mass aqueous solution (202 parts by mass) of polyvinyl alcohol, and then emulsified and dispersed at 20°C to obtain an emulsified liquid having a volume average particle diameter of 1 μm. Further, the obtained emulsified liquid was continuously stirred at 50°C for 4 hours. Further, water was added thereto to adjust the concentration of solid contents, thereby obtaining a 15.9% by mass microcapsule liquid containing a color-forming agent.

<Composition of mixed solution 1>

| | |
|---|---|
| Color-forming agent: 3,3-bis(2-methyl-1-octyl-3-indolyl)phthalide (manufactured by BASF) | 0.7 parts by mass |
| Organic halogen compound: tribromomethylphenylsulfone (manufactured by SUMITOMO SEIKA CHEMICALS CO., LTD.) | 10 parts by mass |
| Aromatic solvent: tricresyl phosphate (manufactured by DAIHACHI CHEMICALINDUSTRY CO., LTD.) | 23 parts by mass |
| Solvent for producing capsule: ethyl acetate (manufactured by SHOWA DENKO K.K.) | 50 parts by mass |
| Light stabilizer: 2,5-bis(1,1,3,3-tetramethylbutyl)hydroquinone (manufactured by TOKYO CHEMICAL INDUSTRY CO., LTD.) | 0.03 parts by mass |
| Polyisocyanate: (adduct of xylylene diisocyanate and trimethylolpropane, product name "TAKENATE D-110N", manufactured by Mitsui Chemicals, Inc., 75% by mass ethyl acetate solution) | 8 parts by mass |

The obtained microcapsule liquid containing a color-forming agent (20 parts by mass), a 6% by mass aqueous solution of polyvinyl alcohol (product name "Denka Size EP-130", manufactured by Denka Company Limited.) (5 parts by mass), glyoxal (manufactured by Daito Chemical Co., Ltd.) (0.05 parts by mass), and a 50% by mass aqueous solution of sodium dodecylbenzenesulfonate (manufactured by DKS Co. Ltd.) (0.09 parts by mass) were mixed with each other to produce a dispersion liquid for forming an ultraviolet-sensing layer (composition for forming an ultraviolet-sensing layer).

The obtained dispersion liquid for forming an ultraviolet-sensing layer was applied onto a white polyethylene terephthalate sheet (product name "CRISPER K1212", manufactured by Toyobo Co., Ltd.) having a thickness of 188 $\mu$m with an amount of liquid applied of 21 mL/m$^2$, and then heated and dried at 105° for 1 minute to produce an ultraviolet-sensing sheet including the support and the ultraviolet-sensing layer. The amount of solid contents of the ultraviolet-sensing layer applied was 3 g/m$^2$. In addition, the film thickness of the ultraviolet-sensing layer was approximately 3 $\mu$m.

[Examples 2 to 14]

Ultraviolet-sensing sheets of Examples 2 to 14 were produced by the same method as in Example 1, except that the type of components, formulation amount, concentration of solid contents, color-forming agent content per unit area of the ultraviolet-sensing layer, and conditions for forming the capsule wall material were changed as shown in Table 1.

The peak surface area proportion X in the sheets of Examples 5, 6, and 12 was 4%, the peak surface area proportion X in the sheet of Example 7 was 6%, and the peak surface area proportion X in the sheet C3 of Comparative Example 2 was 62%. The measuring methods are as described above.

[Comparative Examples 1 and 2]

An ultraviolet-sensing sheet C1 of Comparative Example 1 and an ultraviolet-sensing sheet C2 of Comparative Example 2 were produced by the same method as in Example 1, except that the components and formulations were changed with reference to Example 1 and Comparative Example 1 of JP2014-164125A, and the amount of liquid applied was 88 mL/m$^2$.

[Comparative Examples 3 and 4]

Commercially available UV label (S type, manufactured by NiGK Corporation) and UV scale (L type, manufactured by FUJIFILM Corporation) were used.

Table 1 is shown below.

Each component shown in Table 1 is as follows.

· TCP (tricresyl phosphate, manufactured by DAIHACHI CHEMICAL INDUSTRY CO., LTD., boiling point: 231°C to 255°C, compatible with n-propanol)

· Soybean oil (manufactured by FUJIFILM Wako Pure Chemical Corporation, boiling point: 300°C or higher, compatible with n-propanol)

· SAS-296 (phenylxylylethane, product name "Nisseki Hisol SAS296", manufactured by JX Nippon Oil and Energy Corp, boiling point: 290°C to 305°C, compatible with n-propanol)

· BMPS: tribromomethylphenylsulfone (manufactured by SUMITOMO SEIKA CHEMICALS CO., LTD.)
· Color-forming agent A: 3,3-bis(2-methyl-1-octyl-3-indolyl)phthalide (manufactured by BASF)
Color-forming agent C: 3',6'-bis(diethylamino)-2-(4-nitrophenyl)spiro[isoindole-1,9'-xanthene]-3-one (manufactured by Hodogaya Chemical Co., Ltd.)
· LCV: LEUCO CRYSTAL VIOLET (product name "LCV", manufactured by Yamada Chemical Co., Ltd.)
· BTHQ: 2,5-bis(1,1,3,3-tetramethylbutyl)hydroquinone (manufactured by TOKYO CHEMICAL INDUSTRY CO., LTD.)
· D-110N (adduct of xylylene-1,3-diisocyanate and trimethylolpropane, product name "TAKENATE D-110N", manufactured by Mitsui Chemicals, Inc., 75% by mass ethyl acetate solution)
· D-120N (adduct of hydrogenated xylylene-1,3-diisocyanate and trimethylolpropane, product name "TAKENATE D-120N", manufactured by Mitsui Chemicals, Inc., 75% by mass ethyl acetate solution)
· D-140N (adduct of isophorone diisocyanate and trimethylolpropane, product name "TAKENATE D-140N", manufactured by Mitsui Chemicals, Inc., 75% by mass ethyl acetate solution)
· D-160N (adduct of hexamethylene diisocyanate and trimethylolpropane, product name "TAKENATE D-160N", manufactured by Mitsui Chemicals, Inc., 75% by mass ethyl acetate solution)

In addition, in Table 1, the numerical value in parentheses described together with the component name is intended to be a content (part by mass).
In addition, LCV corresponds to a color-forming agent which forms color by oxidation, and exhibits blue color by oxidation. The color-forming agent A corresponds to a color-forming agent which forms color by action of acid, and exhibits red color by the action of acid.
In addition, in Examples shown in Table 1, the sheets 1 to 14 correspond to the embodiment 1, and the sheets 5 to 7, and 12 correspond to the embodiment 2.
Ethyl acetate, which is the solvent for producing a capsule used in Examples, did not remain in the microcapsule after the ultraviolet-sensing member was produced. In other words, the microcapsule in the ultraviolet-sensing member according to the embodiment of the present invention did not contain the ethyl acetate.

[Measurement and evaluation of optical density]

(Difference in optical density at wavelength of 222 nm)

Using Care 222 (registered trademark), the ultraviolet-sensing member produced in each example was irradiated with light until an irradiation amount of light having a wavelength of 222 nm reached 3 $mJ/cm^2$.
Thereafter, using a spectrophotometer Spectrolino (GretagMacbeth AG), values of yellow optical densities, magenta optical densities, and cyan optical densities were measured with the ultraviolet-sensing member before the light irradiation and the ultraviolet-sensing member after the light irradiation, and in a case where a value of a cyan optical density of the ultraviolet-sensing member before the light irradiation was denoted as C1, a value of a cyan optical density of the ultraviolet-sensing member after the light irradiation was denoted as C2, a value of a yellow optical density of the ultraviolet-sensing member before the light irradiation was denoted as Y1, a value of a yellow optical density of the ultraviolet-sensing member after the light irradiation was denoted as Y2, a value of a magenta optical density of the ultraviolet-sensing member before the light irradiation was denoted as M1, and a value of a magenta optical density of the ultraviolet-sensing member after the light irradiation was denoted as M2, a difference between C1 and C2, a difference between Y1 and Y2, and a difference between M1 and M2 were measured. Among differences in optical density of the difference between C1 and C2, the difference between Y1 and Y2, and the difference between M1 and M2, the largest value is shown in the column of "Difference in optical density at wavelength of 222 nm" in Table 2.

(Evaluation of change in tint)

It was evaluated whether or not it could be determined by a change in tint of the ultraviolet-sensing member in a case where the ultraviolet-sensing member produced in each example was irradiated with light by adjusting the distance and time so that the integrated illuminance was 3 $mJ/cm^2$.

AA: it could be determined very easily.
A: it could be determined easily.
B: it could not be determined easily.

(Evaluation of fogging)

Using a handy UV lamp SLUV-8 (AS ONE Corporation), assuming fogging with a fluorescent lamp, the ultraviolet-sensing member produced in each example was irradiated with light until an irradiation amount of light having a wavelength of 365 nm reached 20 mJ/$cm^2$.

Thereafter, using a spectrophotometer Spectrolino (GretagMacbeth AG), values of yellow optical densities, magenta optical densities, and cyan optical densities were measured with the ultraviolet-sensing member before the light irradiation and the ultraviolet-sensing member after the light irradiation, and in a case where a value of a cyan optical density of the ultraviolet-sensing member before the light irradiation was denoted as C1, a value of a cyan optical density of the ultraviolet-sensing member after the light irradiation was denoted as C2, a value of a yellow optical density of the ultraviolet-sensing member before the light irradiation was denoted as Y1, a value of a yellow optical density of the ultraviolet-sensing member after the light irradiation was denoted as Y2, a value of a magenta optical density of the ultraviolet-sensing member before the light irradiation was denoted as M1, and a value of a magenta optical density of the ultraviolet-sensing member after the light irradiation was denoted as M2, a difference between C1 and C2, a difference between Y1 and Y2, and a difference between M1 and M2 were measured. Among the difference between C1 and C2, the difference between Y1 and Y2, and the difference between M1 and M2, the largest value is shown in the column of "Difference in optical density at wavelength of 365 nm" in Table 2. As the values of the difference between C1 and C2, the difference between Y1 and Y2, and the difference between M1 and M2 described above is smaller, the fogging can be further suppressed. The fogging means that the tint changes due to the influence of light having a wavelength different from that of light having a wavelength to be measured.

In addition, as a difference between the difference in optical density in a case of being irradiated with light at a wavelength of 365 nm and the difference in optical density in a case of being irradiated with light at a wavelength of 222 nm, it is easy to determine whether the amount of irradiation which inactivates the novel coronavirus has been applied, and the fogging can be suppressed.

In Table 1, the column of "Amount of color-forming agent/ultraviolet-sensing layer g/$m^2$" indicates a content (g/$m^2$) of the color-forming agent per unit area of the ultraviolet-sensing layer.

In Table 2, the column of "Color difference ΔE" indicates a color difference ΔE between before the light irradiation and after the light irradiation of the ultraviolet-sensing member of each Examples and Comparative Examples, which were measured according to the method described in the embodiment B above.

In Table 2, the column of "Difference in integrated value" was measured according to the method described in the embodiment C above.

It indicates the difference between the integrated value 1 of the absorbance and the integrated value 2 of the absorbance of the ultraviolet-sensing member of each Examples and Comparative Examples.

In Table 2, the column of "Difference between difference in optical density at 365 nm and difference in optical density at 222 nm" indicates a difference between the difference in optical density, which is shown in the column of "Difference in optical density at wavelength of 365 nm", and the difference in optical density, which is shown in the column of "Difference in optical density at wavelength of 222 nm".

[Table 1]

| | Sheet | Microcapsule | | | | | | | | | | Amount of color-forming agent/ultraviolet-sensing layer g/m² |
|---|---|---|---|---|---|---|---|---|---|---|---|---|
| | | Non-aromatic solvent | Aromatic solvent | | Photoactivator | Color-forming agent | Light stabilizer | Formation of capsule wall material | | Photoactivator/color-forming agent (mass ratio) | Concentration of solid contents of microcapsule liquid (% by mass) | |
| | | Having heteroatom | Having heteroatom | Not having heteroatom | | | | Raw material | Reaction condition | | | |
| Example 1 | Sheet 1 | - | TCP (23) | - | BMPS (10) | Color-forming agent A (0.7) | BTHQ | D-11 ON (8) | 50°C, 4 hours | 14.29 | 15.9 | 0.035 |
| Example 2 | Sheet 2 | Soybean oil (6) | TCP (17) | - | BMPS (10) | Color-forming agent A (2.6) | BTHQ | D-110N (31) | 50°C, 4 hours | 3.85 | 21.2 | 0.139 |
| Example 3 | Sheet 3 | - | TCP (23) | - | BMPS (10) | Color-forming agent A (2.6) | BTHQ | D-110N (31) | 50°C, 4 hours | 3.85 | 21.2 | 0.139 |
| Example 4 | Sheet 4 | - | TCP (17) | SAS-296 (6) | BMPS (10) | Color-forming agent A (2.6) | BTHQ | D-110N (31) | 50°C, 4 hours | 3.85 | 21.2 | 0.139 |
| Example 5 | Sheet 5 | - | TCP (23) | - | BMPS (10) | Color-forming agent A (2.6) | BTHQ | D-120N (31) | 50°C, 8 hours | 3.85 | 21.2 | 0.139 |
| Example 6 | Sheet 6 | - | TCP (17) | SAS-296 (6) | BMPS (10) | Color-forming agent A (2.6) | BTHQ | D-120N (31) | 50°C, 8 hours | 3.85 | 21.2 | 0.139 |
| Example 7 | Sheet 7 | - | TCP (17) | SAS-296 (6) | BMPS (10) | Color-forming agent A (2.6) | BTHQ | D140N/D160N (15.5/15.5) | 50°C, 8 hours | 3.85 | 21.2 | 0.139 |

(continued)

| | Sheet | Microcapsule | | | | | | | | | | Amount of color-forming agent/ ultraviolet-sensing layer $g/m^2$ |
|---|---|---|---|---|---|---|---|---|---|---|---|---|
| | | Non-aromatic solvent | Aromatic solvent | | Photoactivator | Color-forming agent | Light stabilizer | Formation of capsule wall material | | Photoactivator/ color-forming agent (mass ratio) | Concentration of solid contents of microcapsule liquid (% by mass) | |
| | | Having heteroatom | Having heteroatom | Not having heteroatom | | | | Raw material | Reaction condition | | | |
| Example 8 | Sheet 8 | - | TCP (23) | - | BMPS (10) | Color-forming agent A (2.6) | BTHQ | D-110N (8) | 50°C, 4 hours | 3.85 | 16.4 | 0.139 |
| Example 9 | Sheet 9 | - | TCP (23) | - | BMPS (10) | Color-forming agent A (1.3) | BTHQ | D-110N (31) | 50°C, 4 hours | 7.69 | 20.9 | 0.070 |
| Example 10 | Sheet 10 | - | TCP (23) | - | BMPS (10) | Color-forming agent A (0.4) | BTHQ | D-110N (8) | 50°C, 4 hours | 25.00 | 15.8 | 0.023 |
| Example 11 | Sheet 11 | - | TCP (23) | - | BMPS (10) | Color-forming agent A (0.3) | BTHQ | D-110N (8) | 50°C, 4 hours | 33.33 | 15.8 | 0.017 |
| Example 12 | Sheet 12 | - | TCP (23) | - | BMPS (10) | Color-forming agent A (0.7) | BTHQ | D-120N (31) | 50°C, 8 hours | 14.29 | 20.7 | 0.035 |
| Example 13 | Sheet 13 | - | TCP (17) | SAS-296 (6) | BMPS (5) | Color-forming agent A (2.6) | BTHQ | D-110N (31) | 50°C, 4 hours | 1.92 | 21.2 | 0.139 |
| Example 14 | Sheet 14 | - | TCP (23) | SAS-296 (7) | BMPS (2.7) | Color-forming agent A (2.6) | BTHQ | D-110N (31) | 50°C, 4 hours | 1.04 | 21.4 | 0.140 |

(continued)

| | Sheet | Microcapsule | | | | | | | | | | Amount of color-forming agent/ultraviolet-sensing layer g/$m^2$ |
|---|---|---|---|---|---|---|---|---|---|---|---|---|
| | | Non-aromatic solvent | Aromatic solvent | | Photoactivator | Color-forming agent | Light stabilizer | Formation of capsule wall material | | Photoactivator/color-forming agent (mass ratio) | Concentration of solid contents of microcapsule liquid (% by mass) | |
| | | Having heteroatom | Having heteroatom | Not having heteroatom | | | | Raw material | Reaction condition | | | |
| Comparative Example 1 | Sheet C1 | - | TCP (24) | - | BMPS (1.5) | LCV (3.0) | BTHQ | D-110N (24) | 40°C, 3 hours | 0.50 | 23.0 | 0.583 |
| Comparative Example 2 | Sheet C2 | - | TCP (24) | - | BMPS (1.5) | LCV (3.0) | BTHQ | D-120N (24) | 40°C, 3 hours | 0.50 | 23.0 | 0.583 |

[Table 2]

| | Sheet | Color-forming characteristics | | | | Evaluation of change in tint | Evaluation of fogging | | |
|---|---|---|---|---|---|---|---|---|---|
| | | Difference in optical density at wavelength of 222 nm | | Color difference ΔE | Difference in integrated value | Determination | Difference in optical density at wavelength of 365 nm | | Difference between difference in optical density at 365 nm and difference in optical density at 222 nm |
| Example 1 | Sheet 1 | 0.35 | Difference between M1 and M2 | 44.4 | 37.1 | AA | 0.04 | Difference between M1 and M2 | 0.31 |
| Example 2 | Sheet 2 | 0.21 | Difference between M1 and M2 | 27.1 | 21.5 | A | 0.10 | Difference between M1 and M2 | 0.11 |
| Example 3 | Sheet 3 | 0.24 | Difference between M1 and M2 | 33.4 | 24.8 | A | 0.17 | Difference between M1 and M2 | 0.07 |
| Example 4 | Sheet 4 | 0.24 | Difference between M1 and M2 | 31.4 | 24.8 | A | 0.15 | Difference between M1 and M2 | 009 |
| Example 5 | Sheet 5 | 0.29 | Difference between M1 and M2 | 38.0 | 30.4 | AA | 0.05 | Difference between M1 and M2 | 0.24 |
| Example 6 | Sheet 6 | 0.33 | Difference between M1 and M2 | 42.0 | 34.9 | AA | 0.08 | Difference between M1 and M2 | 0.25 |
| Example 7 | Sheet 7 | 0.29 | Difference between M1 and M2 | 37.6 | 30.4 | AA | 0.05 | Difference between M1 and M2 | 0.24 |
| Example 8 | Sheet 8 | 0.36 | Difference between M1 and M2 | 44.9 | 38.2 | AA | 0.14 | Difference between M1 and M2 | 0.22 |
| Example 9 | Sheet 9 | 0.23 | Difference between M1 and M2 | 33.8 | 23.7 | A | 0.04 | Difference between M1 and M2 | 0.19 |

(continued)

| | Sheet | Color-forming characteristics | | | | Evaluation of change in tint | Evaluation of fogging | | |
|---|---|---|---|---|---|---|---|---|---|
| | | Difference in optical density at wavelength of 222 nm | | Color difference ΔE | Difference in integrated value | Determination | Difference in optical density at wavelength of 365 nm | | Difference between difference in optical density at 365 nm and difference in optical density at 222 nm |
| Example 10 | Sheet 10 | 0.29 | Difference between M1 and M2 | 38.3 | 30.4 | AA | 0.02 | Difference between M1 and M2 | 0.27 |
| Example 11 | Sheet 11 | 0.24 | Difference between M1 and M2 | 32.6 | 24.8 | A | 0.01 | Difference between M1 and M2 | 0.23 |
| Example 12 | Sheet 12 | 0.36 | Difference between M1 and M2 | 45.7 | 38.2 | AA | 0.07 | Difference between M1 and M2 | 0.29 |
| Example 13 | Sheet 13 | 0.21 | Difference between M1 and M2 | 28.1 | 21.5 | A | 0.05 | Difference between M1 and M2 | 0.16 |
| Example 14 | Sheet 14 | 0.20 | Difference between M1 and M2 | 25.0 | 20.4 | A | 0.03 | Difference between M1 and M2 | 0.17 |
| Comparative Example 1 | Sheet C1 | 0.03 | Difference between C1 and C2 | 3.6 | 5.2 | B | 0.03 | Difference between C1 and C2 | 0.00 |
| Comparative Example 2 | Sheet C2 | 0.04 | Difference between C1 and C2 | 3.8 | 7.1 | B | 0.02 | Difference between C1 and C2 | 002 |
| Comparative Example 3 | UV label | 0.13 | Difference between M1 and M2 | 16.9 | 12.6 | B | 0.05 | Difference between M1 and M2 | 0.08 |
| Comparative Example 4 | UV scale | 0.03 | Difference between C1 and C2 | 4.4 | 5.2 | B | 0.02 | Difference between C1 and C2 | 0.01 |

As shown in Table 2, it was confirmed that the ultraviolet-sensing member according to the embodiment of the present invention exhibited a desired effect.

From the comparison between Examples and Comparative Examples, it was confirmed that, in a case where the ultraviolet-sensing member had a photoactivator and the mass ratio of the content of the photoactivator to the content of the color-forming agent was more than 1.0, the determination could be easily performed.

In addition, from the comparison of Examples 1, 8, 10 and 11, it was confirmed that, in a case where the mass ratio of the content of the photoactivator to the content of the color-forming agent was 8.00 or more and 30.00 or less, the determination could be easily performed and the fogging at a wavelength of 365 nm could be suppressed.

In addition, from the comparison between Examples 4, 6, 7, 12, and Comparison Example 2, it was confirmed that, in a case where the ultraviolet-sensing member contained a microcapsule containing a photoactivator, a color-forming agent, and a solvent, a capsule wall of the microcapsule contained one or more resins selected from the group consisting of a polyurea having an aliphatic ring, a polyurethane urea having an aliphatic ring, and a polyurethane having an aliphatic ring, and the peak surface area proportion X obtained by the peak surface area proportion calculation method X was 30% or less, the determination could be easily performed and the fogging at a wavelength of 365 nm could be suppressed.

In addition, from the comparison between Examples and Comparative Examples, it was confirmed that, in a case where the content of the color-forming agent in the ultraviolet-sensing layer was 0.14 $g/m^2$ or less per unit area of the ultraviolet-sensing layer, the determination could be easily performed.

In addition, from the comparison between Examples and Comparative Examples, it was confirmed that, in a case where the photoactivator was a photoacid generator and the color-forming agent was a color-forming agent which forms color by action of acid, the effect of the present invention was more excellent.

<Examples 15 to 16>

Ultraviolet-sensing sheets of Examples 15 and 16 were produced by the same method as in Example 1, except that the type of components, formulation amount, concentration of solid contents, color-forming agent content per unit area of the ultraviolet-sensing layer, and conditions for forming the capsule wall material were changed as shown in Table 3.

<Example 17>

Polyvinyl butyral (PVB) (50 parts by mass), tetrahydrofuran (300 parts by mass), and ethanol (68.2 parts by mass) were mixed to dissolve the polymer. Tribromomethylphenylsulfone (manufactured by SUMITOMO SEIKA CHEMICALS CO., LTD.) (BMPS) (10.0 parts by mass) and 3,3-bis(2-methyl-1-octyl-3-indolyl)phthalide (manufactured by BASF) (color-forming agent A) (5.0 parts by mass) were added to the obtained polymer solution to obtain a solution for forming an ultraviolet-sensing layer. The obtained solution for forming an ultraviolet-sensing layer was applied onto a white polyethylene terephthalate sheet (product name "CRISPER K1212", manufactured by Toyobo Co., Ltd.) having a thickness of 188 μm so that a film thickness after drying was 5 μm, and then dried to produce an ultraviolet-sensing sheet including the support and the ultraviolet-sensing layer.

<Examples 18 to 21>

Ultraviolet-sensing sheets of Examples 18 to 21 were produced by the same method as in Example 17, except that the formulation amount and color-forming agent content per unit area of the ultraviolet-sensing layer were changed as shown in Table 4.

[Table 3]

| | Sheet | Microcapsule | | | | | | | | Amount of color-forming agent/ ultraviolet-sensing layer g/m$^{2-}$ |
|---|---|---|---|---|---|---|---|---|---|---|
| | | Solvent | Photoactivator | Color-forming agent | Light stabilizer | Formation of microcapsule wall material | | Photoactivator/color-forming agent (mass ratio) | Concentration of solid contents of microcapsule liquid (% by mass) | |
| | | | | | | Raw material | Reaction condition | | | |
| Example 15 | Sheet 15 | TCP (23) | BMPS (10) | Color-forming agent C (0.7) | BTHQ | D-110N (8) | 50°C, 4 hours | 14.29 | 15.9 | 0.035 |
| Example 16 | Sheet 16 | TCP (23) | BMPS (10) | Color-forming agent A (0.7) | BTHQ | D-110N/D-120N (8) | 50°C, 8 hours | 14.29 | 15.9 | 0.035$^{i}$ |

[Table 4]

| | Sheet | Ultraviolet-sensing layer | | | | Amount of color-forming agent/ ultraviolet-sensing layer g/m$^2$ |
|---|---|---|---|---|---|---|
| | | Binder | Photoactivator | Color-forming agent | Photoactivator/co lor-forming agent (mass ratio) | |
| Example 17 | Sheet 17 | PVB (50) | BMPS (10.0) | Color-forming agent A (5.0) | 2.00 | 0.500 |
| Example 18 | Sheet 18 | PVB (50) | BMPS (5.0) | Color-forming agent A (4.0) | 1.25 | 0.400 |
| Example 19 | Sheet 19 | PVB (50) | BMPS (10.0) | Color-forming agent A (2.0) | 5.00 | 0.200 |
| Example 20 | Sheet 20 | PVB (50) | BMPS (10.0) | Color-forming agent A (1.0) | 10.00 | 0.100 |
| Example 21 | Sheet 21 | PVB (50) | BMPS (2.0) | Color-forming agent A (1.0) | 2.00 | 0.100 |

In addition, in Tables 3 and 4, the numerical value in parentheses described together with the component name is intended to be a content (part by mass).

The color-forming agent C corresponds to a color-forming agent which forms color by action of acid, and exhibits red color by the action of acid.

The above-described evaluations were performed on the ultraviolet-sensing sheets produced above. The results are summarized in Table 5.

[Table 5]

| | Sheet | Color-forming characteristics | | | | Evaluation of change in tint | Evaluation of fogging | | |
|---|---|---|---|---|---|---|---|---|---|
| | | Difference in optical density at wavelength of 222 nm | | Color difference $\Delta E$ | Difference in integrated value | Determination | Difference in optical density at wavelength of 365 nm | | Difference between difference in optical density at 365 nm and difference in optical density at 222 nm |
| Example 15 | Sheet 15 | 0.20 | Difference between M1 and M2 | 20.7 | 19.9 | A | 0.15 | Difference between M1 and M2 | 0.05 |
| Example 16 | Sheet 16 | 0.36 | Difference between M1 and M2 | 44.8 | 38.0 | AA | 0.05 | Difference between M1 and M2 | 0.31 |
| Example 17 | Sheet 17 | 0.25 | Difference between M1 and M2 | 32.5 | 25.9 | A | 0.05 | Difference between M1 and M2 | 0.20 |
| Example 18 | Sheet 18 | 0.23 | Difference between M1 and M2 | 31.1 | 23.7 | A | 0.05 | Difference between M1 and M2 | 0.18 |
| Example 19 | Sheet 19 | 0.29 | Difference between M1 and M2 | 35.5 | 30.4 | AA | 0.05 | Difference between M1 and M2 | 0.24 |
| Example 20 | Sheet 20 | 0.30 | Difference between M1 and M2 | 37.3 | 31.5 | AA | 0.05 | Difference between M1 and M2 | 0.25 |
| Example 21 | Sheet 21 | 0.28 | Difference between M1 and M2 | 37.8 | 29.3 | AA | 0.05 | Difference between M1 and M2 | 0.23 |

As shown in Table 5, it was confirmed that the ultraviolet-sensing member according to the embodiment of the present invention exhibited a desired effect.

Explanation of References

10: ultraviolet-sensing member

12: support

14: ultraviolet-sensing layer

## Claims

**1.** An ultraviolet-sensing member,

wherein, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 mJ/cm$^2$,

using a spectrophotometer Spectrolino (GretagMacbeth AG), values of yellow optical densities, magenta optical densities, and cyan optical densities are measured with the ultraviolet-sensing member before the light irradiation and the ultraviolet-sensing member after the light irradiation, and
a value of a cyan optical density of the ultraviolet-sensing member before the light irradiation is denoted as C1, a value of a cyan optical density of the ultraviolet-sensing member after the light irradiation is denoted as C2, a value of a yellow optical density of the ultraviolet-sensing member before the light irradiation is denoted as Y1, a value of a yellow optical density of the ultraviolet-sensing member after the light irradiation is denoted as Y2, a value of a magenta optical density of the ultraviolet-sensing member before the light irradiation is denoted as M1, and a value of a magenta optical density of the ultraviolet-sensing member after the light irradiation is denoted as M2,

any one of a difference between C1 and C2, a difference between Y1 and Y2, or a difference between M1 and M2 is 0.20 or more.

**2.** An ultraviolet-sensing member,
wherein, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 mJ/cm$^2$, a color difference $\Delta E$ between before the light irradiation and after the light irradiation is 20.0 or more.

**3.** An ultraviolet-sensing member,

wherein, in a case where, using a KrCl excimer lamp as a light source, the ultraviolet-sensing member is irradiated with light through a filter which substantially shields light having a wavelength of 230 to 300 nm until an irradiation amount of light having a wavelength of 222 nm reaches 3 mJ/cm$^2$, a difference between an integrated value 1 of an absorbance of the ultraviolet-sensing member before the light irradiation in a wavelength range of 450 to 700 nm, which is obtained by a method 1, and an integrated value 2 of an absorbance of the ultraviolet-sensing member after the light irradiation in the wavelength range of 450 to 700 nm, which is obtained by a method 2, is 18.0 or more,
the method 1: a reflection spectrum of the ultraviolet-sensing member before the light irradiation is measured to obtain a reflection spectrum in which a horizontal axis is a wavelength and a vertical axis is an absorbance, and then the integrated value 1 of the absorbance is obtained by integrating the absorbance for each 1 nm from a wavelength of 450 nm to a wavelength of 700 nm in the reflection spectrum
the method 2: a reflection spectrum of the ultraviolet-sensing member after the light irradiation is measured to obtain a reflection spectrum in which a horizontal axis is a wavelength and a vertical axis is an absorbance, and then the integrated value 2 of the absorbance is obtained by integrating the absorbance for each 1 nm from

a wavelength of 450 nm to a wavelength of 700 nm in the reflection spectrum.

**4.** The ultraviolet-sensing member according to any one of claims 1 to 3, wherein the ultraviolet-sensing member is a sheet-like ultraviolet-sensing member.

**5.** The ultraviolet-sensing member according to any one of claims 1 to 4, wherein the ultraviolet-sensing member includes an ultraviolet-sensing layer containing a color-forming agent.

**6.** The ultraviolet-sensing member according to claim 5, wherein a content of the color-forming agent in the ultraviolet-sensing layer is 0.140 $g/m^2$ or less per unit area of the ultraviolet-sensing layer.

**7.** The ultraviolet-sensing member according to claim 5 or 6,

wherein the color-forming agent is selected from the group consisting of a color-forming agent which forms color by being oxidized and a color-forming agent which forms color by action of acid, and
the ultraviolet-sensing layer contains at least one photoactivator selected from the group consisting of a photooxidant and a photoacid generator.

**8.** The ultraviolet-sensing member according to claim 7,

wherein the color-forming agent is a color-forming agent which forms color by action of acid,
the ultraviolet-sensing layer contains a photoacid generator, and
a mass ratio of a content of the photoactivator to a content of the color-forming agent is more than 1.00.

**9.** The ultraviolet-sensing member according to claim 7 or 8,

wherein the photoactivator includes a compound represented by General Formula (6),

$$R^3\text{-}L^1\text{-}CX^3X^4X^5 \qquad (6)$$

in General Formula (6), $R^3$ represents an aryl group which may have a substituent or a heteroaryl group which may have a substituent, $L^1$ represents -SO- or $-SO_2-$, and $X^3$, $X^4$, and $X^5$ each independently represent a hydrogen atom or a halogen atom, where all of $X^3$, $X^4$, and $X^5$ are not hydrogen atoms at the same time.

**10.** The ultraviolet-sensing member according to claim 7 or 8, wherein the color-forming agent includes any one structure selected from the group consisting of a lactone ring, a lactam ring, a sultone ring, a sultine ring, a ring-opened body of these rings, and an azobenzene structure.

**11.** The ultraviolet-sensing member according to any one of claims 1 to 6, wherein the ultraviolet-sensing member includes an ultraviolet-sensing layer containing a microcapsule which contains a photoactivator, a color-forming agent, and a solvent having a heteroatom.

**12.** The ultraviolet-sensing member according to claim 11,

wherein a capsule wall of the microcapsule contains one or more resins selected from the group consisting of a polyurea having an aliphatic ring, a polyurethane urea having an aliphatic ring, and a polyurethane having an aliphatic ring, and
a peak surface area proportion X obtained by the following peak surface area proportion calculation method X is 30% or less,
the peak surface area proportion calculation method X: a liquid chromatography measurement is performed for each of a first solution which is obtained by cutting out two test pieces of the same size from the ultraviolet-sensing member and immersing one of the test pieces in n-propanol for 7 days and a second solution obtained by immersing the other test piece in n-propanol for 1 hour, and a proportion of a peak area of the color-forming agent in the second solution to a peak area of the color-forming agent in the first solution is calculated as the peak surface area proportion X.

**13.** An ultraviolet-sensing kit comprising:

the ultraviolet-sensing member according to any one of claims 1 to 12.

FIG. 1

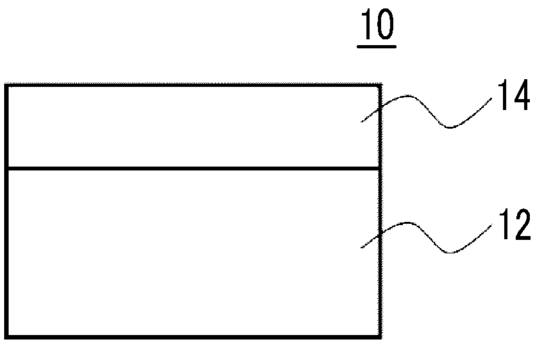

## INTERNATIONAL SEARCH REPORT

International application No.
**PCT/JP2022/004340**

**A. CLASSIFICATION OF SUBJECT MATTER**

***C09K 9/02***(2006.01)i; ***G01J 1/50***(2006.01)i
FI: G01J1/50; C09K9/02 B

According to International Patent Classification (IPC) or to both national classification and IPC

**B. FIELDS SEARCHED**

Minimum documentation searched (classification system followed by classification symbols)

G01J1/48-1/52; C09K9/00-9/02; C09K3/00; B01J13/02-13/22; G03C1/72-1/735; G03F7/105

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2016/017701 A1 (FUJIFILM CORP.) 04 February 2016 (2016-02-04) paragraphs [0001], [0002], [0027], [0029], [0031]-[0035], [0039], [0042], [0046], [0051], [0064], [0110], [0111] | 1-13 |
| Y | JP 60-89352 A (MITSUI TOATSU KAGAKU KK) 20 May 1985 (1985-05-20) page 1, right column, lines 1-12, page 3, lower left column, line 4 from the bottom to lower right column, line 1, page 7, upper left column, lines 6-7 | 1-13 |
| Y | JP 2019-187727 A (USHIO INC.) 31 October 2019 (2019-10-31) paragraphs [0009], [0037] | 1-13 |
| Y | JP 2018-517488 A (THE TR. OF COLUMBIA UNIVERSITY IN THE CITY OF NEW YORK) 05 July 2018 (2018-07-05) claim 34, paragraphs [0005], [0034] | 1-13 |
| A | WO 2014/129642 A1 (FUJIFILM CORP.) 28 August 2014 (2014-08-28) paragraph [0016] | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 April 2022** | **19 April 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/004340**

| Patent document cited in search report | Publication date (day/month/year) | Patent family member(s) | Publication date (day/month/year) |
|---|---|---|---|
| WO 2016/017701 A1 | 04 February 2016 | US 2017/0131144 A1<br>paragraphs [0003], [0005], [0094], [0097], [0100]-[0105], [0110], [0117], [0128], [0138], [0162], [0163], [0267]<br>CN 106537103 A | |
| JP 60-89352 A | 20 May 1985 | (Family: none) | |
| JP 2019-187727 A | 31 October 2019 | US 2019/0321499 A1<br>paragraphs [0009], [0037]<br>CN 110393857 A | |
| JP 2018-517488 A | 05 July 2018 | WO 2016/196904 A1<br>claim 34, paragraphs [0005], [0054]<br>CN 107613895 A<br>EP 3302328 A1<br>US 2018/0169279 A1<br>US 2020/0085984 A1<br>US 2020/0215215 A1<br>US 2020/0306397 A1<br>CN 111888514 A<br>US 2020/0353112 A1<br>JP 2021-90828 A<br>JP 2021-90829 A | |
| WO 2014/129642 A1 | 28 August 2014 | US 2015/0355021 A1<br>paragraph [0051]<br>US 2017/0089760 A1<br>JP 2014-186031 A<br>JP 2017-167155 A<br>CN 105074400 A<br>CN 107091689 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015191001 A
- WO 2017158943 A
- US 3445234 A
- JP 5257272 A
- JP H5257272 A
- WO 2009008248 A
- US 3282693 A
- US 3615568 A
- JP 62039728 B
- JP S62039728 B
- WO 2016017701 A
- US 3552973 A
- JP 62161860 A
- JP S62161860 A
- JP 61067034 A
- JP S6167034 A
- JP 62050382 A
- JP S62050382 A
- JP 59190886 A
- JP S59190886 A
- JP 60242094 A
- JP S60242094 A
- JP 60049991 A
- JP S60049991 A
- US 3281383 A
- US 3773695 A
- US 3793268 A
- JP 48040347 B
- JP S48040347 B
- JP 49024159 B
- JP S49024159 B
- JP 48080191 A
- JP S48080191 A
- JP 48084086 B
- JP S48084086 B
- WO 2019159570 A
- US 3726804 A
- US 3796696 A
- JP 2017167155 A
- JP 1207741 A
- JP H1207741 A
- JP 2004233614 A
- JP 2014164125 A


### Non-patent literature cited in the description


- **HIROKI, KITAGAWA et al.** Effectiveness of 222-nm ultraviolet light on disinfecting SARS-CoV-2 surface contamination. *American Journal of Infection Control, https://www.sciencedirect.com/science/article/pii/S0196655320308099*
- The Lecture Summary. *Spring Meeting of the Society of Photographic Science and Technology of Japan,* 1968, 55
- Polyurethane Resin Handbook. Nikkan Kogyo Shimbun, 1987